# EUROPÄISCHE PATENTANMELDUNG

(11) **EP 1 004 598 A2**
(43) Veröffentlichungstag der Anmeldung: **31.05.2000**
(21) Anmeldenummer: 99118541.4
(22) Anmeldetag: 18.09.1999
(51) Int. Cl.: C07K 17/00, A61M 1/36

(54) **Beschichtete Trägermaterialien für Blut-, Plasma- Gewebewäsche und mit diesen Trägermaterialien ausgerüstete Säulen**

(30) Priorität: 01.10.1998 DE 19845286
(71) Anmelder: PD Dr. Dunzendorfer, 60528 Frankfurt/M (DE)
(72) Erfinder: PD Dr. Dunzendorfer, 60528 Frankfurt/M (DE)

(57) **Zusammenfassung**

Es werden Trägermaterialien für den Einsatz bei der Blut-, Plasma- oder Gewebewäsche beschrieben, umfassend eine Beschichtung aus den jeweiligen Antikörpern gegen TNF und/oder gegen Anti-TNF und/oder gegen Fragmente von TNF und/oder gegen Fragmente von Anti-TNF oder gegen TNF-Transportproteine, wobei die Beschichtung weitere Antikörper umfaßt, die gegen spezifische Krankheitserreger wirksam sind, sowie mit solchen beschichteten Trägermaterialien ausgerüsteten Säulen zur Blut-, Plasma- oder Gewebewäsche.

## Beschreibung

Die Erfindung betrifft beschichtete Trägermaterialien, die für die Blut- bzw. Plasmawäsche eingesetzt werden können, wobei die Trägermaterialien eine Beschichtung aus Antikörpern gegen Tumor Nekrose Faktor, dessen Präkursoren, Metaboliten und Transportproteinen tragen.

Therapeutische Extrakorporalsysteme werden in der Regel bei der Plasmapherese oder bei der Dialyse eingesetzt und sind bei Erkrankungen mit humoralen Substanzen als technisch gut entwickelte Systeme bekannt (Sprenger, KBG: In Franz, HE (ed), Blutreinigungsverfahren, Stuttgart 1985). Die Abtrennung von krankmachenden Plasmafaktoren, die mit dem erkrankten Gewebe in einem Gleichgewicht stehen, hat sich bei seltenen Erkrankungen, wie der IgM-Paraproteinämie, dem systemischen Krankheitsbild des Lupus erythematodes und dem Goodpasture Syndrom bewährt. Hohlfasermembranen mit Mikroporen von 3-4 mio. Dalton separieren das Plasma, nachdem zuvor die korpuskulären Bestandteile des Blutes entfernt worden sind. Wichtig ist der Ersatz des abgetrennten Plasmas durch eiweißhaltige Lösungen, wie 5% Humanalbumin. Im speziellen wird man versuchen, gezielt die krankmachenden Substanzen, wie Autoantikörper, Immunkomplexe, eiweißgebundene Toxine und Paraproteine zu entfernen. Es gilt für einen Austausch von ca. 3-4 l Plasma gegen eine Substitutionslösung, daß sich dabei die Ausgangskonzentration des Proteins auf 37% verringert. Mit den folgenden Austauschprozessen nimmt die Effektivität des Verfahrens immer weiter ab (22%, 14%, 8%) (Schröder, JO, Euler HH: Plasmapherese bei Autoimmunerkrankungen, Gelbe Hefte 4/95). Da zwischen dem Extrazellulärraum und dem Plasma ein Fließgleichgewicht besteht, können die ausgetauschten Plasmafaktoren innerhalb von 24 h schnell wieder auf 68,5% des Ausgangswertes ansteigen (Prinzip der Rückverteilung).

Außerdem wird durch den Entzug von Antikörpern die Biosynthese kurzfristig gesteigert, so daß man von einem raschen Wiederanstieg über 68,5% des Ausgangswertes nach Plasmapherese heute nicht mehr überrascht ist (Schröder, JO, Euler HH: Plasmapherese bei Autoimmunerkrankungen, Gelbe Hefte 4/95).

Benutzt man dagegen Säulen, die mit speziellen Antikörpern beschichtet sind, in dem Verfahren der Plasmapherese, so werden nur die krankmachenden Faktoren aus dem Plasma entfernt, alle anderen Plasmabestandteile werden unverändert zurückgeführt, so daß komplizierte Ergänzungen von lebenswichtigen Plasmafaktoren, wie bei der allgemeinen Plasmapherese üblich, in der Regel entfallen. Das Behandlungsverfahren für chronisch Kranke läßt sich dadurch wesentlich vereinfachen .

Bei einer Vielzahl von chronischen Erkrankungen werden im Gewebe und im Plasma konstante Veränderungen gemessen. So werden beispielsweise bei der Erkrankung der chronischen rheumatoiden Arthritis Interleukin 1 (IL-1) und der Tumor Nekrose Faktor alpha in der entzündeten Gelenksynovia von Makrophagen gebildet.

Da bei chronischen Entzündungen ein Netzwerk von beteiligten Zellen und Stoffwechselprodukten vorliegt, erscheinen noch weitere meßbare Faktoren, wie Interleukin 6 (IL-6), Interferon alpha (IFNa), Zellwachstum stimulierende und inhibierende Faktoren.

Neben dieser Gruppe der sogenannten proinflammatorischen Zytokinen gibt es noch drei weitere Zytokiningruppen, die immunmodulatorisch, chemotaktisch und stoffwechselaktiv bei allen chronischen Erkrankungen wirken (Feldmann, M., Brennan, FM., Maini R.: Role of Cytokines in Rheumatoid Arthritis, Ann. Rev. Immunol., 14, 1996).

Wichtig sind dabei die Wachstumsfaktoren aus Blutplättchen (PFDG) und aus Fibroblasten (FGF) sowie der transforming growth factor (TGF β). In den verschiedenen Stadien der chronischen Entzündungen wechseln sich aktive und weniger aktive Phasen ab, weitere Faktoren treten auf und interagieren mit positiven und negativen Effekten, aber die Voraussetzung für die gesamten Prozesse scheint eine deutliche Erhöhung des Tumor Nekrose Faktors zu sein (Feldmann, M., Elliot, MJ., Woody, JN., Maini, R.: Anti-Tumor Necrosis Factor - a Therapy of Rheumatoid Arthritis, Adv. Immunol., 64, 1997).

Bei einer Langzeittherapie mit Fremdeiweißen muß mit der Bildung von Antikörpern gerechnet werden, so daß nach Jahren der Anwendung ein Wirkverlust der medikamentösen Therapie mit monoklonalen Antikörpern festzustellen ist.

Aus der EP 0 214 392 B1 ist ein medizinisches Gerät bekannt, insbesondere ein Filter, eine Kanüle, ein Katheter oder Implantant aus Kunststoff oder kunststoffbeschichtetem Metall oder Glas zur Fixierung krankheitserregender Keime, insbesondere Viren, Bakterien und Pilzen, sowie pathogener Stoffwechselprodukte, Toxine, Fettkörper und Arzneimittel, bei dem die Kunststoffoberfläche mit fest daran gebundenen oder kovalent damit verbundenen Antikörpern ausgestattet ist, dadurch gekennzeichnet, daß es sich bei den Antikörpern handelt um die F(ab)₂-Teile, vorzugsweise die durch Affinitätschromatographie nachgereinigten F(ab)₂-Teile, von homologen oder monoklonalen Immunglobulinen der Klassen G1, G2, G3 und/oder G4 und/oder IGM, IGE, IGD und/oder IGA, selektiv wirksam sind gegen die spezifischen Antigen oder antigenen Determinanten der jeweiligen krankheitserregenden Keime, insbesondere Viren, Bakterien und Pilze, sowie der phathogenen Stoffwechselprodukte, Toxine, Fettkörper und Arzneimittel.

Der Erfindung liegt die Aufgabe zugrunde, beschichtete Trägermaterialien für die Blut-, Plasma- oder Gewebewäsche sowie mit diesen beschichteten Trägermaterialien ausgerüsteten Säulen oder Gewebe verträgliche Implantate zu schaffen, so daß bei der Plasmapherese oder Gewebeinkubation gezielt die krankmachenden Faktoren aus dem Plasma oder erkranktem Gewebe entfernt werden, jedoch alle übrigen Plasma-Bestandteile unverändert zurückgeführt werden können oder die übrigen Gewebebestandteile unverändert bleiben , so daß komplizierte Ergänzungen von lebenswichtigen Plasmafaktoren, wie bei der herkömmlich angewandten Plasmapherese oder diffuse chronische Gewebeveränderungen , entfallen.

Diese Aufgabe wird durch ein Trägermaterial gelöst, umfassend eine Beschichtung aus den jeweiligen Antikörpern gegen TNF und/oder gegen Anti-TNF und/oder gegen Fragmente von TNF und/oder gegen Fragmente von Anti-TNF oder gegen TNF-Transportproteine, wobei die Beschichtung weitere Antikörper umfaßt, die gegen spezifische Krankheitserreger wirksam sind.

Die Unteransprüche 1 bis 156 geben vorteilhafte Ausgestaltungen solch eines Trägermaterials wieder.

Dieses Trägermaterial findet vorzugsweise in einer Säule gemäß den Ansprüchen 157 bis 159 Verwendung.

Die Ansprüche 160 bis 163 geben den bevorzugten Einsatz solcher Säulen an.

Die Verwendung des erfindungsgemäßen beschichteten Trägermaterials sowie der Säulen in Extrakorporalsystemen führt zu einer verbesserten Effektivität dieser Extrakorporalsysteme mit günstigen Therapieeffekten bei akuten und chronischen Erkrankungen, Durchblutungsstörungen, Krebsleiden und Abstoßungskrisen bei Organtransplantationen.

Reduziert man TNF im Plasma und über das bestehende Fließgleichgewicht ebenfalls in chronisch erkrankten Gewebe durch Plasmapherese mit monoklonal beschichteten Antikörper-Säulen, ist deren Langzeitanwendung ohne Wirkverlust möglich. Zusätzlich lassen sich dadurch die für die chronischen Erkrankungen notwendigen Medikamente in der Dosierung reduzieren. Auf ähnliche Weise lassen sich die erfindungsgemäßen beschichteten Trägermaterialien und Säulen, die mit diesen Trägermaterialien ausgerüstet sind, bei Organtransplantationen mit Abstoßungskrisen, Dialyse-Patienten, bestimmten Krebserkrankungen usw. einsetzen.

Insbesondere werden die Säulen im Extrakorporalverfahren als Medikalprodukte bei Menschen mit chronischen oder wiederkehrenden Erkrankungen und meßbarem Tumor Nekrose Faktor, Anti Tumor Nekrose Faktor, Anti Tumor Nekrose Faktor Gen-Konstrukt, Tumor Nekrose Faktor Präkursor - Protein, Tumor Nekrose Faktor Bindungsprotein, im Blut oder Gewebe, eingesetzt, und die als konstante Beschichtung den Antikörper gegen Tumor Nekrose Faktor oder gegen Anti Human Tumor Nekrose Faktor oder gegen Anti Tumor Nekrose Gen-Konstrukt oder gegen Anti Tumor Nekrose Faktor Präkursor oder gegen Tumor Nekrose Faktor Transportprotein oder gegen Tumor Nekrose Faktor Transportprotein Antikörper sowie weitere Antikörper gegen spezifische Krankheiten tragen. Der Einsatz dieser Extrakorporalsysteme verbessert bei akuten und chronischen Erkrankungen, Krebsleiden, Durchblutungsstörungen und Abstoßungskristen nach Organtransplantation den Therapieerfolg.

Die Entfernung der krankmachenden Noxen erfolgt dabei durch Überleiten von Blut über das erfindungsgemäße Trägermaterial, welches eine Beschichtung zum einen aus Antikörpern gegen TNF und/oder gegen Anti-TNF und/oder gegen Fragmente von TNF und/oder gegen Fragmente von Anti-TNF umfaßt, wobei das Trägermaterial in der Beschichtung zusätzlich weitere Antikörper enthält, die gegen spezifische Krankheitserreger wirksam sind, so daß beim Überleiten von Blut bzw. Plasma über das Trägermaterial die fest bzw. kovalent gebundenen Antikörper mit den Antigenen eine spezifische feste Antikörper-Antigen-Bindung eingehen, und die Antigene auf diese Weise aus dem Blut bzw. Plasma abgetrennt werden.

Vorzugsweise findet das erfindungsgemäße Trägermaterial Verwendung als Beschichtungsmittel für Säulen zur Blut- bzw. Plasmareinigung, oder das erfindungsgemäße Trägermaterial kann in Form von Kügelchen oder eines Granulats in Säulen gepackt werden, wobei jedoch der Fachmann weiß, daß das erfindungsgemäße Trägermaterial auch für andere medizinische Geräte x-beliebiger geometrischer Form verwendet werden kann, beispielsweise auch für Filter, Kanülen, Katheter, Implantate usw.

Vorzugsweise besteht der Träger für das Trägermaterial aus Kunststoffen, kunststoffbeschichteten Metallen, Celluloseverbindungen, Stärke- und Stärkeaggregaten, Sepharose und Sepharose-Derivaten, mit und ohne vorbereitete Gruppen zur kovalenten Bindung.

Als Kunststoffe können vorzugsweise Polyolefin, Polyester, Polyether, Polyamid, Polyimid, Polyurethan, Polyvinylchlorid, Polystyrol, Polysulfon, Polyacrylat, Polymethacrylat, Polypropylen, Polyvinylpyrrolidon, ein Fluorpolymeres, ein Derivat dieser Verbindungen oder eine Mischung oder ein Copolymeres davon oder ein Silicon-Kautschuk eingesetzt werden.

Vorzugsweise besitzen diese Kunststoffe polare Eigenschaften oder werden mittels einer chemischen Behandlung polar gemacht.

Vorzugsweise wird zur Bindung der Antikörper an den Träger ein Haftvermittler, wie Bromcyan, Thiophosgen und Thionylchlorid, eingesetzt.

Vorzugsweise sind die Antikörper mittels Adsorption oder kovalent auf der Trägeroberfläche gebunden. Vorzugsweise sind die F_{c}-, F_{(ab)2}- oder F_{ab}-Teile der Antikörper an die Trägeroberfläche gebunden.

Vorzugsweise enthält die Beschichtung des erfindungsgemäßen Trägermaterials 1-95 Gew.-% Antikörper gegen TNF und/oder gegen Anti-TNF und/oder gegen Fragmente von TNF und/oder gegen Fragmente von Anti-TNF, und die Restmenge auf 100 Gew.-% wird von weiteren Antikörpern gebildet, die gegen spezifische Krankheitserreger wirksam sind.

Bei einer anderen bevorzugten Ausführungsform des erfindungsgemäßen Trägermaterials enthält die Beschichtung 1-95 Gew.-% Antikörper gegen TNF und die Restmenge auf 100 Gew.-% wird durch weitere Antikörper gebildet, die gegen spezifische Krankheitserreger wirksam sind.

Bei einer weiteren bevorzugten Ausführungsform kann die Beschichtung des Trägermaterials auch zu 100 Gew.-% aus Antikörpern gegen TNF bestehen.

Vorzugsweise wird das erfindungsgemäße Trägermaterial in einer Säule für die Blut- bzw. Plasmawäsche verwendet, wobei die Säule mit dem Trägermaterial beschichtet oder damit gefüllt sein kann.

Solche Säulen lassen sich bei allen Erkrankungen einsetzen, bei denen spezifische Erreger, spezifische Wachstumsfaktoren, spezifische Proteine oder Peptide, spezifische Toxine, spezifische Enzyme, spezifische Hormone, spezifische Rezeptoren, spezifische Zell- oder Gewebemarker, spezifische Zytokinine in biologischen Flüssigkeiten, in Geweben und in Zellkulturen von Geweben nachzuweisen sind und zu denen monoklonale Antikörper in definierter Weise herstellbar sind, und deren Antigene oder Antikörper sich zur Beschichtung von extrakorporalen Säulensystemen eignen, sowie
bei allen Erkrankungen, bei denen spezifische Erreger, spezifische Wachstumsfaktoren, spezifische Proteine und Peptide, spezifische Toxine, spezifische Enzyme, spezifische Hormone, spezifische Rezeptoren, spezifische Zell- oder Gewebemarker, spezifische Zytokinine in biologischen Flüssigkeiten, in Geweben und in Zellkulturen von Geweben nicht nachzuweisen sind, aber im Blut und/oder im Gewebe oder in Zellkulturen von Geweben definierte Antigene und/oder Antikörper, die im direkten oder indirektem Zusammenhang mit den Erkrankungen stehen, meßbar sind, und deren Antigene oder monoklonale Antikörper sich zur Beschichtung von extrakorporalen Säulensystemen eignen, sowie
bei allen Erkrankungen, bei denen direkt oder indirekt ein Hinweis auf immunogene Prozesse vorliegt und Antigene und/oder Antikörper nachweisbar sind, und deren Antigene oder monoklonale Antikörper sich zur Beschichtung von extrakorporalen Säulensystemen eignen, sowie
bei allen Erkrankungen, die andere ursächliche Gründe als die unter Anspruch 158 bis 160 genannten vorliegen, und Isolate aus biologischen Flüssigkeiten von erkrankten Organen sich als Antigene oder monoklonale und/oder polyklonale Antikörper zur Beschichtung oder Füllung von Säulen eignen.

Ein Verfahren zum Aufbringen der Antikörper auf das erfindungsgemäße Trägermaterial ist beispielsweise in EP 0 214 392 B1 beschrieben, wobei vor dem Auftragen der Antikörper beispielsweise die Kunststoffoberfläche mittels γ-Strahlen, Ionenätzung, Ozonätzung oder mit einer Lösung von einem pH-wert von 7,5 bis 10,5 oder mit Acrolein vorbehandelt wird, um die Haftfestigkeit der Antikörper an der Oberfläche zu verbessern. Die Behandlung wird je nach Kunststoffausgangsmaterial bei einem pH-Wert von 7,5 bis 10,5 durchgeführt, wobei die Reaktionszeit ebenfalls in Abhängigkeit vom Kunststoff 5 bis 10 Minuten beträgt. Die Aufrauhung der zu beschichtenden Kunstsstoffoberfläche kann insbesondere auch durch Alkylierungsbehandlung mit Methyliodid vorgenommen werden.

Zusätzlich zu der Aufrauhung der Oberfläche kann der Kunststoff auch noch mit einem Haftvermittler, insbesondere Bromcyan, Thiophosgen oder Thionylchlorid, behandelt werden, der ebenfalls die Haftung der Antikörper an der Kunststoffoberfläche bewirkt, insbesondere durch Ausbildung einer kovalenten Bindung.

Eine weitere Möglichkeit zum Aufbringen der Antikörper auf das erfindungsgemäße Trägermaterial erfolgt durch Tränken der durch entsprechende Aufrauhungsbehandlung vorbehandelten Kunststoffoberfläche mit den gesättigten Antikörperlösungen. Das Tränken der Kunststoffoberfläche erfolgt über einen Zeitraum von 1 bis 10 Stunden bei etwa 20 bis 40°C.

Nach der Bindung mittels Haftvermittler oder Adsorption der Antikörper auf der Trägeroberfläche werden die Trägermaterialien beispielsweise mit einer Phosphat-Pufferlösung gewaschen, um überschüssige Antikörper abzuspülen. Danach werden die Materialien bei beispielsweise einer Temperatur von 20 bis 40°C für 8 bis 24 Stunden getrocknet und anschließend steril verpackt.

Bei verschiedenen experimentellen Modellen wurde die Wirkung monoklonal-beschichteter Anti-TNF-Säulen bzw. mehrfach beschichteter Säulen überprüft.

Tabelle 1 zeigt die Ergebnisse für die Therapie der Pyelonephritis mit AK-TNFa-Plasmapherese.

Bei jeweils 10 Hunden wurde experimentell eine Coli Pyelonephritis erzeugt, die mit underdosierten Antibiotika für 4 Tage behandelt wurde. Nach 21 Tagen bei bestehenden Entzündungszeichen (Leukzyturie; Erythtrozyturie/Urinkeimtest positiv für E. Coli) ergaben sich für TNF alpha deutlich erhöhte Werte gegenüber den Vorwerten. Die mit Anti-TNF alpha beladenen Säulen wurden jeden zweiten Tag für jeweils 4 Stunden angeschlossen. Nach 14 Tagen waren in den Nierengewebeproben der kranken Tiere (T), bei denen eine Blutwäsche durchgeführt wurde, deutliche Unterschiede zu den unbehandelten Tieren (K) der Kontrollgruppe festzustellen, wobei der TNF alpha-Spiegel im Plasma auf leicht erhöhte ursprüngliche Ausgangswerte zurückgegangen war.

Eine ebenso deutliche Besserung zeigte die Urinkultur, wobei bei den behandelten Tieren (T) noch 10³ Colonien/ml gegenüber 10⁶ Colonien/ml bei den unbehandelten Tieren festzustellen war, und sich auch der Gewebebefund für die behandelten pathalogischen Tiere im Vergleich zu den unbehandelten Tieren der Kontrollgruppe wesentlich verbesserte.

Eine weitere Verbesserung der Resultate bei der Therapie der Pyelonephritis läßt sich erzielen, wenn die Beschichtung der Säule zusätzlich zu den Antikörpern gegen TNFa noch Antikörper gegen Protein A enthält. Hier zeigten sich insbesondere verbesserte Werte beim Urinkeimtest am Ende der Behandlungsdauer.

**TABELLE 1**

| **THERAPIE DER PYELONEPHRITIS MIT AK-TNFa-PLASMAPHERESE** | | | | | |
|---|---|---|---|---|---|
| (Tag) | TNFa (Plasma) (µg) (T/K) | | Urinkultur (C/ml) (T/K) | | Gewebebefund (path.-T/K) |
| 1 | 0,20+/-0,05 | 0,18+/-0,04 | 10⁵/ | 10⁵ | -/- |
| 10 | 0,36+/-0,06 | 0,32+/-0,05 | 10⁵/ | 10⁶ | ++/++ |
| 21 | 0,45+/-0,05 | 0,43+/-0,06 | 10⁶ | 10⁷ | ++++/++++ |
| 35 | 0,25+/-0,06 | 0,52+/-0,06 | 10⁴ | 10⁷ | ++/++++ |
| 49 | 0,26-/+0,05 | 0,56+/-0,05 | 10³ | 10⁶ | ++/+++++ |

| **THERAPIE DER PYELONEPHRITIS MIT AK-TNFa-/PROTEIN-A-PLASMAPHERESE** | | | | | |
|---|---|---|---|---|---|
| (Tag) | TNFa(Plasma)(µg) (T/K) | | Urinkultur (C/ml) (T/K) | | Gewebebefund (path.-T/K) |
| 1 | 0,18+/-0,07 | 0,19+/-0,04 | 10⁵/ | 10⁵ | +/+ |
| 10 | 0,39+/-0,06 | 0,36+/-0,07 | 10⁵/ | 10⁵ | ++/++ |
| 21 | 0,55+/-0,05 | 0,53+/-0,06 | 10⁶ | 10⁶ | ++++/++++ |
| 35 | 0,22+/-0,06 | 0,52+/-0,06 | 10⁴ | 10⁷ | ++/++++ |
| 49 | 0,26-/+0,05 | 0,56+/-0,05 | 10² | 10⁶ | ++/+++++ |
| Versuchsansatz 1: Erzeugt wurde eine chronische Pyelonephritis (Inokulationspyelonephritis) durch eine insuffiziente Antibiotikatheraple, der chronische Verlauf ist in der Kontrollgruppe zu erkennen (K). Die Gewebehistologie (Nierenpunktionen) zeigt eine ausgeprägte lymphozytäre Infiltration, die in der behandelten Gruppe deutlich geringer ist. Im Versuch 2 ergaben sich durch den Zusatz des Anti Protein A-Faktors günstigere Verläufe der chronischen Erkrankung. C = Colonien | | | | | |

Tabelle 2 zeigt die Ergebnisse bei der Therapie der Colitis bei der AK-TNFa-Plasmapherese, wobei die Säule eine Beschichtung mit Antikörpern (AK) gegen Tumor Nekrose Faktor alpha (TNF alpha) aufweist. Auch hier zeigte sich bei den erkrankten Tieren bei Behandlung mittels der Anti-TNFa-Säule nach 14 Tagen eine Verbesserung im Vergleich zu den unbehandelten Tieren.

Eine weitere Verbesserung ergab sich bei Behandlung mit einer Säule, die neben Antikörpern gegen TNF alpha noch zusätzlich Antikörper gegen Protein A enthielt.

**TABELLE 2**

| **THERAPIE DER COLITIS MIT AK-TNFa-PLASMAPHERESE** | | | | |
|---|---|---|---|---|
| (Tag) | TNFa(Plasma) (T/K) | | Durchfälle (path.) (T/K) | Gewebebefund (path.-T/K) |
| 1 | 0,20+/-0,05 | 0,17+/-0,04 | +/0 | +/+ |
| 15 | 0,37+/-0,05 | 0,35+/-0,06 | +++/+++ | +++/+++ |
| 31 | 0,28+/-0,05 | 0,46+/-0,06 | +/+++ | ++/++++ |

| **THERAPIE DER COLITIS MIT AK-TNFa-/PROTEIN-A-PLASMAPHERESE** | | | | |
|---|---|---|---|---|
| (Tag) | TNFa (Plasma) (µg) (T/K) | | Durchfälle (Path) (T/K) | Gewebebefund (path.-T/K) |
| 1 | 0,18+/-0,07 | 0,19+/-0,04 | +/+ | +/+ |
| 15 | 0,43+/-0,06 | 0,46+/-0,07 | +++/+++ | +++/+++ |
| 31 | 0,20+/-0,05 | 0,53+/-0,06 | +/++++ | +/++++ |
| Versuchsansatz 1: Erzeugt wurde eine Colitis (5% Dextransulfat im Trinkwasser für 6 Tage), der chronische Verlauf ist in der Kontrollgruppe zu erkennen (K). Die Gewebehistologie zeigt eine ausgeprägte lymphozytäre Infiltration, die in der behandelten Gruppe deutlich geringer ist. Im Versuch 2 ergaben sich durch den Zusatz des Anti Protein A-Faktors günstigere Verläufe der chronischen Erkrankung. | | | | |

Tabelle 3 zeigt die Resultate bei der Therapie der rheumatoiden Arthritis mit AK-TNFa-Plasmapherese, bei welcher eine Säule mit einer Beschichtung aus Antikörpern gegen TNF alpha eingesetzt wurde.

Hier ergab sich für die behandelten Patienten eine deutliche Verbesserung des TNF alpha-Spiegels, der Response-Rate als auch des prozentualen Abfalls an C-reaktivem Protein.

Eine weitere Verbesserung läßt sich durch Verwendung einer Säule erzielen, bei welcher neben Antikörpern gegen TNF alpha auch in der Beschichtung Antikörper gegen Protein-A/beta-Streptococcus-A enthalten sind.

Die Behandlung mittels Antikörper gegen TNF alpha und gegen beta-hämolyserende Streptokokken und AK-Protein wurde für zunächst 2 Wochen innerhalb von 6 Wochen bei insgesamt 12 Wochen Behandlungsdauer durchgeführt. Die Werte für TNF alpha gingen rapide zurück und die Beweglichkeit verbesserte sich auffällig.

**TABELLE 3**

| **THERAPIE DER RHEUMATOIDEN ARTHRITIS MIT AK-TNFa-PLASMAPHERESE** | | | | |
|---|---|---|---|---|
| (Tag) | TNFa(Plasma) (µg) (P/K) | | RESPONSE RATE (%P/K) | CRP (% Abfall-P/K) |
| 1 | 0,456+/-0,054 | 0,348+/-0,044 | 0/0 | 5/4 |
| 14 | 0,332+/-0,062 | 0,342+/-0,053 | 35/8 | 34/0 |
| 42 | 0,226+/-0,053 | 0,343+/-0,063 | 48/10 | 46/0 |
| 56 | 0,222+/-0,063 | 0,352+/-0,063 | 54/9 | 47/1 |
| 84 | 0,180-/+0,053 | 0,346+/-0,053 | 51/10 | 88/2 |

| **THERAPIE DER RHEUMATOIDEN ARTHIRITIS MIT AK-TNFa-/PROTEIN-A/β-STREKTOCCOCUS-A-PLASMAPHERESE** | | | | |
|---|---|---|---|---|
| (Tag) | TNFa (Plasma) (µg) (P/K) | | RESPONSE RATE (%P/K) | CRP (% Abfall-P/K) |
| 1 | 0,518+/-0,075 | 0,619+/-0,046 | 0/0 | 0/0 |
| 14 | 0,339+/-0,064 | 0,636+/-0,076 | 37/6 | 34/0 |
| 42 | 0,355+/-0,053 | 0,653+/-0,066 | 39/4 | 43/7 |
| 56 | 0,212+/-0,064 | 0,552+/-0,064 | 54/9 | 65/16 |
| 84 | 0,096-/+0,054 | 0,526+/-0,053 | 59/11 | 87/18 |
| Behandelt wurden Patienten mit chronischer rheumatoider Arthritis, die unter der Standardtherapie mit Methotrexat-Kortison sich weiter verschlechterten. Die Therapie mit Anti-TNF in der Plasmapherese besserte den Zustand. Der Entzündungswert im Plasma, gemessen als CRP-Spiegel, ging zurück. Im zweiten Behandlungsprotokoll wurden die Säulesysteme mehrfach beschichtet und noch günstigere klinische Daten durch die Anti-TNF/beta-hämolysierende Streptokokken-Säule erreicht. Kollektive n=7 CRP = C-reaktives Protein P = behandelte Patienten K = unbehandelte Patienten | | | | |

Die Resultate bei der Therapie der Multiplen Sklerose mit AK-TNFa-Plasmapherese sind in Tabelle 4 angegeben, wobei sich bei Patienten mit fortschreitender Multipler Sklerose eine günstige Beeinflussung der TNF alpha-Werte ergab, wenn die Blutwäsche mit einer Säule durchgeführt wurde, deren Beschichtung allein aus Antikörpern gegen den Tumor Nekrose Faktor bestand.

Werden zusätzlich in die Beschichtung der Säule noch Antikörper gegen Protein-A/beta-2-Microglobulin eingeführt, dann ergibt sich eine noch signifikantere Verbesserung der TNF alpha-Werte, der Response-Rate und des Abfalls für das C-reaktive Protein bei den behandelten Patienten.

**TABELLE 4**

| **THERAPIE DER MULTIPLEN SKLEROSE MIT AK-TNFa-PLASMAPHERESE** | | | | |
|---|---|---|---|---|
| (Tag) | TNFa (Plasma) (µg) (P/K) | | RESPONSE RATE (%P/K) | CRP (% Abfall P/K) |
| 1 | 0,348+/-0,063 | 0,338+/-0,054 | 0/0 | 85/78 |
| 14 | 0,235+/-0,052 | 0,419+/-0,064 | 31/7 | 44/76 |
| 42 | 0,230+/-0,053 | 0,435+/-0,065 | 39/9 | 42/70 |
| 56 | 0,176+/-0,063 | 0,450+/-0,074 | 45/9 | 39/73 |
| 84 | 0,151-/+0,053 | 0,346+/-0,054 | 45/10 | 38/72 |

| **THERAPIE DER MULTIPLEN SKLEROSE MIT AK-TNFa-/PROTEIN-A/BETA-2-MICROGLOBULIN-PLASMAPHERESE** | | | | |
|---|---|---|---|---|
| (Tag) | TNFa(Plasma)(µg) (P/K) | | RESPONSE RATE (%P/K) | CRP (% Abfall P/K) |
| 1 | 0,447+/-0,054 | 0,445+/-0,042 | 0/0 | 85/78 |
| 14 | 0,227+/-0,063 | 0,446+/-0,054 | 45/10 | 32/72 |
| 42 | 0,225+/-0,043 | 0,446+/-0,063 | 48/10 | 31/70 |
| 56 | 0,123+/-0,063 | 0,354+/-0,062 | 52/11 | 28/69 |
| 84 | 0,087-/+0,042 | 0,347+/-0,055 | 59/10 | 23/72 |
| Patienten mit multipler Sklerose unter Progress wurden 4 Wochen nach Absetzen aller Medikamente mit den Anti-TNF-Säulen im 3-wöchigen Zyklus behandelt. Die Kontrollgruppe erhielt weiter die Standardtherapie mit Interferon bei bekanntem progressiven Status. Der Plasmaspiegel von TNF und CRP wurde bestimmt, daneben die sog. Response Rate festgelegt nach den Kriterien subjektiver Befindlichkeit, der Beweglichkeit und dem allgemeinen Karnosfki Index. Im zweiten Therapieversuch wurde die Säule mehrfach beschichtet mit Antikörpern gegen TNF/B-2M. Hierbei konnte noch ein günstigerer Verlauf beobachtet werden. Kollektive n = 8 Patienten | | | | |

Die Resultate bei der Therapie der chronischen Glomerulitis mit AK-TNFa-Plasmapherese sind in Tabelle 5 angegeben.

Hierbei wurden Patienten mit chronisch fortschreitender Nierenerkrankung (progressive Glomerulonephritis, IGA GN) krankhaftem Plasma- und Urinbefund mit der Plasmapherese-Säulentechnik unter Verwendung einer mit Antikörpern gegen TNF beschichteten Säule in 3-wöchigen Abständen behandelt. Der pathalogische Urinbefund ging synchron mit dem Plasma-TNFa-Wert nach 7 Wochen zurück, und noch günstigere Ergebnisse bei der chronischen Glumerulonephritis ließen sich mit einer Säule erreichen, bei welcher die Beschichtung sowohl Antikörper gegen TNF als auch Antikörper gegen beta-2-Microglobulin enthielt.

**TABELLE 5**

| **THERAPIE DER CHRONISCHEN GLOMERULITIS MIT AK-TNFa-PLASMAPHERESE** | | | | |
|---|---|---|---|---|
| (Tag) | TNFa(Plasma) (µg) (P/K) | | RESPONSE RATE (%P/K) | PROTEINURIE (% Abfall-P/K) |
| 1 | 0,75+/-0,043 | 0,86+/-0,032 | 0/0 | 5/8 |
| 14 | 0,58+/-0,034 | 0,74+/-0,032 | 33/7 | 34/6 |
| 42 | 0,52+/-0,024 | 0,64+/-0,022 | 39/9 | 46/7 |
| 56 | 0,48+/-0,022 | 0,71+/-0,034 | 41/9 | 49/7 |
| 84 | 0,36-/+0,015 | 0,65+/-0,035 | 41/10 | 54/7 |

| **THERAPIE DER CHRONISCHEN GLOMERULITIS MIT AK-TNFa-/A-BETA-2-MICROGLOBULIN-PLASMAPHERESE** | | | | |
|---|---|---|---|---|
| (Tag) | TNFa(Plasma) (µg) (P/K) | | RESPONSE RATE (%P/K) | PROTEINURIE (% Abfall-P/K) |
| 1 | 0,75+/-0,035 | 0,85+/-0,045 | 0/0 | 5/8 |
| 14 | 0,71+/-0,035 | 0,76+/-0,037 | 44/9 | 42/7 |
| 42 | 0,55+/-0,031 | 0,75+/-0,027 | 48/10 | 46/7 |
| 56 | 0,52+/-0,022 | 0,67+/-0,035 | 57/11 | 58/9 |
| 84 | 0,42-/+0,022 | 0,75+/-0,041 | 59/10 | 58/7 |
| Patienten mit chronischer progressiver Glomerulonephritis wurden 4 Wochen nach Absetzen aller Medikamente mit den Anti-TNF-Säulen im 3-wöchigen Zyklus behandelt. Die Kontrollgruppe erhielt weiter die Standardtherapie mit Imunorek bei bekanntem progressiven Status. Der Plasmaspiegel von TNF a wurde bestimmt ebenso wie das Ausmaß der Proteinurie bzw. der Rückgang der Proteinurie, daneben die sog. Response Rate festgelegt nach den Kriterien der Befindlichkeit, der Normalisierung des Blutdruckes. Im zweiten Therapieversuch wurde die Säule mehrfach beschichtet mit Antikörpern gegen TNF/B2M. Hierbei konnte noch ein günstigerer Verlauf beobachtet werden. Kollektive n = 8 Patienten | | | | |

Die Resultate bei der Therapie der chronischen Hepatitis B mittels AK-TNFa-Plasmapherese und einer kombinierten AK-TNFa-/HbsAg-Plasmapherese sind in Tabelle 6 angegeben.

Nach viermaliger Säulenapplikation zeigte sich nach 12 Wochen eine Normalisierung der chronisch erhöhten Laborwerte, wobei bei Verwendung der Säule mit Antikörpern sowohl gegen TNFa als auch HbsAg (Hepatitis-B Surface-Antigen) eine weitere Verbesserung erreicht werden kann.

**TABELLE 6**

| **THERAPIE DER CHRONISCHEN HEPATITIS B MIT AK-TNFa-PLASMAPHERESE** | | | | |
|---|---|---|---|---|
| (Tag) | TNFa(plasma) (µg) (P/K) | | RESPONSE RATE (%P/K) | Anti HBc, HbsAg (% Abfall-P/K) |
| 1 | 0,197+/-0,042 | 0,184+/-0,041 | 0/0 | 5/8 |
| 14 | 0,192+/-0,043 | 0,179+/-0,042 | 33/6 | 32/6 |
| 42 | 0,155+/-0,032 | 0,174+/-0,042 | 39/9 | 38/7 |
| 56 | 0,078+/-0,023 | 0,183+/-0,034 | 44/8 | 49/7 |
| 84 | 0,076-/+0,037 | 0,157+/-0,027 | 46/9 | 54/7 |

| **THERAPIE DER CHRONISCHEN HEPATITIS B MIT AK-TNFa-/HbsAg-PLASMAPHERESE** | | | | |
|---|---|---|---|---|
| (Tag) | TNFa(Plasma) (µg) (P/K) | | RESPONSE RATE (%P/K) | Anti HBc, Anti HbsAg (% Abfall-P/K) |
| 1 | 0,177+/-0,03 | 0,168+/-0,047 | 0/0 | 5/8 |
| 14 | 0,173+/-0,035 | 0,161+/-0,054 | 44/9 | 42/7 |
| 42 | 0,060+/-0,024 | 0,158+/-0,035 | 48/10 | 46/7 |
| 56 | 0,053+/-0,034 | 0,157+/-0,036 | 56/10 | 58/9 |
| 84 | 0,064-/+0,025 | 0,155+/-0,055 | 59/10 | 63/7 |
| Patienten mit chronischer progressiver Hepatitis wurden 4 Wochen nach Absetzen aller Medikamenten mit den Anti-TNF-Säulen im 3-wöchigen Zyklus behandelt. Die Kontrollgruppe erhielt weiter die Standardtherapie mit Interferon bei bekanntem progressiven Status. Der Plasmaspiegel von TNF a wurde bestimmt ebenso wie das Ausmaß der Hepatitisserologie bzw. der Rückgang der Proteinurie, daneben die sog. Response Rate, festgelegt nach den Kriterien der Befindlichkeit, der Normalisierung der Leberenzyme. Im zweiten Therapieversuch wurde die Säule mehrfach beschichtet mit Antikörpern gegen Hepatitis-B surface Antigen (HbsAg) und HBc = Hepatitis B core (Kern). Hierbei konnte noch ein günstigerer Verlauf beobachtet werden. Kollektive n = 8 Patienten | | | | |

Die Resultate bei der Therapie der Abstoßungskrise nach Nierentransplantation mit Antikörper-TNFa-Plasmapherese und mittels AK-TNFa-/HLA, OKT3 und Leukozyten-Plasmapherese sind in Tabelle 7 angegeben.

Auch in diesem Fall ergeben sich weitere Verbesserungen bezüglich der TNFa-Werte, der Response-Rate als auch des prozentualen Kreatininabfalls, wenn die Säule zusätzlich zu den Antikörpern gegen TNFa noch mit Antikörpern gegen HLA, OCT3 und Leukozyten ausgerüstet ist.

**TABELLE 7**

| **THERAPIE DER ABSTOSSUNGSKRISE NACH NIERENTRANSPLANTATION MIT AK-TNFa-PLASMAPHERESE** | | | | |
|---|---|---|---|---|
| (Tag) | TNFa(Plasma) (µg) (P/K) | | RESPONSE RATE (%P/K) | KREATININ 0 (% Abfall-P/K) |
| 1 | 0,67+/-0,06 | 0,66+/-0,05 | 0/0 | 5/8 |
| 14 | 0,42+/-0,05 | 0,54+/-0,06 | 35/28 | 30/21 |
| 42 | 0,38+/-0,05 | 0,51+/-0,08 | 39/31 | 38/25 |
| 56 | 0,36+/-0,04 | 0,47+/-0,07 | 44/41 | 46/30 |
| 84 | 0,32-/+0,05 | 0,49+/-0,04 | 47/38 | 52/32 |

| **THERAPIE DER ABSTOSSUNGSKRISE NACH NIERENTRANSPLANTATION MIT AK-TNFa-/HLA,OKT3 UND LEUKOZYTEN-PLASMAPHERESE** | | | | |
|---|---|---|---|---|
| (Tag) | TNFa(plasma) (µg) (P/K) | | RESPONSE RATE (%P/K) | KREATININ I (% Abfall-P/K) |
| 1 | 0,67+/-0,04 | 0,66+/-0,04 | 0/0 | 5/7 |
| 14 | 0,31+/-0,05 | 0,46+/-0,05 | 44/29 | 42/27 |
| 42 | 0,24+/-0,03 | 0,48+/-0,06 | 52/27 | 46/25 |
| 56 | 0,21+/-0,05 | 0,47+/-0,06 | 56/28 | 58/28 |
| 84 | 0,21-/+0,04 | 0,38+/-0,05 | 59/39 | 63/37 |
| Patienten mit Abstossungskrise nach Nierentransplantation wurden in einwöchigem Zyklus behandelt. Die Kontrollgruppe erhielt weiter die Standardtherapie mit Cyclosporin/Ganciclovir/Methyl- prednisolon bei bekanntem progressiven Status. Der Plasmaspoiegel TNF a wurde bestimmt ebenso wie das Ausmaß des Kreatininanstiegs, daneben wurden die sog. Response Rate festgelegt nach den Kriterien der Befindlichkeit, der Normalisierung der Proteinurie. Im zweiten Therapieversuch wurde die Säule mehrfach beschichtet mit Antikörpern gegen HLA,OKT3, monoklonalen Antikörpern gegen Leukozyten. Hierbei konnte noch ein günstigeren Verlauf beobachtet werden. Kollektive n = 5 Patienten | | | | |

Die Resultate bei der Therapie der chronischen Bronchitis mit AK-TNFA--Plasmapherese und mit AK-TNFA-/Cold Agglutin-Plasmapherese sind in Tabelle 8 dargestellt.

Hier wurden Patienten mit chronischer Bronchitis bei erhöhten Plasma-TNF-Werten mittels Anti-TNF-Säulen für 8 Wochen behandelt. Die klinischen Beschwerden gingen nach der sechsten Woche deutlich zurück, und die Atemkapazität wurde deutlich gesteigert. Bei Verwendung einer Säule, deren Beschichtung aus Antikörpern gegen TNF und gegen Cold Agglutinin besteht, ergaben sich bei der Therapie bereits nach 4 Wochen Besserungen.

**TABELLE 8**

| **THERAPIE DER CHRONISCHEN BRONCHITIS MIT AK-TNFa-PLASMAPHERESE** | | | | |
|---|---|---|---|---|
| (Tag) | TNFa(plasma) (µg) (P/K) | | RESPONSE RATE (%P/K) | PROTEINURIE (% Abfall P/K) |
| 1 | 0,295+/-0,043 | 0,86+/-0,032 | 0/0 | 5/8 |
| 14 | 0,278+/-0,034 | 0,178+/-0,032 | 33/7 | 34/6 |
| 42 | 0,262+/-0,024 | 0,162+/-0,022 | 39/9 | 42/7 |
| 56 | 0,158+/-0,022 | 0,177+/-0,034 | 41/9 | 49/7 |
| 84 | 0,149-/+0,015 | 0,230+/-0,035 | 41/10 | 54/7 |

| **THERAPIE DER CHRONISCHEN BRONCHITIS MIT AK-TNFa-/COLD AGGLUTININ-PLASMAPHERESE** | | | | |
|---|---|---|---|---|
| (Tag) | TNFa(Plasma) (µg) (P/K) | | RESPONSE RATE (%P/K) | PROTEINURIE (%, Abfall P/K) |
| 1 | 0,175+/-0,035 | 0,185+/-0,045 | 0/0 | 5/8 |
| 14 | 0,178+/-0,035 | 0,186+/-0,034 | 44/9 | 42/7 |
| 42 | 0,135+/-0,031 | 0,175+/-0,027 | 48/10 | 46/7 |
| 56 | 0,097+/-0,022 | 0,142+/-0,032 | 57/11 | 58/9 |
| 84 | 0,082-/+0,022 | 0,152+/-0,041 | 59/10 | 58/7 |
| Patienten mit chronischer Bronchitis wurden 2 Wochen nach Absetzen aller Medikamente mit der Anti-TNF-Säule im 3-wöchigen Zyklus behandelt. Die Kontrollgruppe erhielt weiter die Standardtherapie mit Antibiotika/Prednisolon bei bekanntem progressiven Status. Der Plasmaspiegel von TNF a wurde bestimmt ebenso wie das Ausmaß der Lungenfunktion. Daneben wurde die sog. Response Rate festgelegt nach den Kriterien der Belastbarkeit. Im zweiten Therapieversuch wurde die Säule mehrfach beschichtet mit Antikörpern gegen TNF/COLD Agglutinin. Hierbei konnte ein günstigerer Verlauf beobachtet werden. Kollektive n = 8 Patienten | | | | |

Im folgenden werden Beschichtungen für das erfindungsgemäße Trägermaterial angegeben, wobei die Beschichtung neben Antikörpern gegen TNF und/oder Anti-TNF und/oder gegen Fragmente von TNF und/oder gegen Fragmente von Anti-TNF noch weitere Antikörper in der Beschichtung enthält, so daß sich erfindungsgemäße Trägermaterialien ergeben, die weitere Antikörper gegen
- ein Bakterien/Virusgemisch, bestehend aus Staphylococcus aureus, Streptococcus mitis, Haemophilus influenza, Diploccocus pneumoniae, Kiebsiella pneunoniae und ozeanae, Staphylococcus pyogenses und viridans, Newisseria cateralis, E. Coli, β-hämolisierende Streptococcus, Hepatitis A Antigen und Hbs Antigen,
- ein Bakterien/Virus-Gemisch, bestehend aus Staphylococcus aureus, Streptococcus mitis, Haemophilus influenza, Diplococcus pneumoniae, Klebsiella pneumoniae und ozeanae, Staphylococcus pyogenses und viridans, Neisseria cateralis, E. Coli und β-hämolisierende Streptococcus,
- die Ribosomen der Bakterien, bestehend aus Staphylococcus aureus, Streptococcus mitis, Haemophilus influenza, Diplococcus pneumoniae, Klebsiella pneumoniae und ozeanae, Staphylococcus pyogenses und viridans, Neisseria cateralis, E. Coli und β-hämolisierende Streptococcus, sowie Antikörper gegen Hepatitis A Antigen, Hbs Antigen,
- die Ribosomen der Bakterien, bestehend aus Styphylococcus aureus, Streptococcus mitis, Haemophilus influenza, Diploccocus pneumoniae, Klebsiella pneumoniae und ozeanae, Staphylococcus pyogenses und viridans, Neisseria cateraus, E. Coli und β-hämolisierende Streptococcus,
- β-hämolysierende Streptokokken A und gegen Virus Capside,
- Polysaccharide von Streptococcus pneumoniae und Virus Capside, Chiamydien und Mykóplasmen,
- Anti-I-cold Agglutinin, Polysaccharide von Streptococcus pneumoniae und Virus Capside, Chiamydien und Mykóplasmen,
- Polysaccharide von Streptococcus pneumoniae und Antikörper gegen Influenzaviren, Influenzaviren, Influenza Hämagglutinin Peptide, Hämagglutinin Peptide,
- Polysaccharide von Streptococcus pneumoniae und Antikörper gegen Influenzaviren, Influenza Hämagglutinin Peptide sowie Anti-I-cold Agglutinin,
- ein Bakterien/Virus-Gemisch, bestehend aus Staphyloccocus aureus, Streptococcus mitis, Haemophilus influenza, Diplococcus pneumoniae, Klebsiella pneumoniae und ozeanae, Staphylococcus pyogenses und viridans, Neisseria cateralis, E. Coii, β-hämolisierende Streptococcus A, Hepatitis A Antigen, Hbs Antigen, Influenzaviren und Influenza Hämagglutinin Peptide sowie Protein A,
- die reverse Transskriptase, Hepatitis A Antigen und Virus Capside,
- die reverse Transskriptase, Hepatitis B Antigen, (Hbs) und Virus Capside,
- die reverse Transskriptase, gegen Hepatitis B Antigen, (Hbs) und das Transaktivator Hbx Protein,
- die reverse Transskriptase und Hepatitis C Virus Genom (Nukleocapsid Protein),
- die reverse Transskriptase, Hepatitis C Antigen, Protein A, Herpes, Influenzaviren und Influenza Hämaggintinin Peptide,
- β-hämolysierende Streptokokken, Endothelin (VEGF), (VEGF-Fragmente), (VEGF-Rezeptor Antagonisten), VCAM, Protein A, Conductin-Protein, SDF-1 Protein, 6-C-Kine-Protein und MIP-3β-Protein,
- β-hämolysierende Streptokokken, Protein A, P-Glykoprotein und Lipide,
- HLA-DR 1,2,4, Endothelin (VEGF), (VEGF-Fragmente), (VEGF-Rezeptor Antagonisten), VCAM, Protein A, Conductin-Protein, SDF-1-Protein, 6-C-Kino-Protein und MIP-3β-Protein,
- HLA-DR 1,2,4, Virus Capside und Oligodendrozyten Glykoprotein als Antikörper vorn monoklonalen Typ (MOG),
- HLA-DR 1,2,4, Oligodendrozyten Glykoprotein (MOG) und das Epstein-Barr-Virus (EBV) als Antikörper vom monoklonalen Typ,
- erkranktes Organgewebe,
- erkranktes Schilddrüsengewebe, gegen Anti Dystrophin und gegen Acetylcholin,
- β-hämolysierende Streptokokken, Influenzaviren, Influenza Hämagglutinin Peptide, Lipopolysaccharide und Chaperonin,
- β-hämolysierende Streptokokken, Van Glykopeptide, Influenzaviren, Influenza Hämagglutinin Peptide, Lipopolysaccharide gegen Matrix-Proteine und beta-2-Mikroglobulin,
- Kreatinin, Harnsäure, Harnstoff und Lipide,
- Cocksackie Viren, Virus Capside und Van-Glykopeptide,
- Herpes, Influenzaviren, Influenza Hämagglutinin Peptide, Cytomegalie Viren, Protein A, Van Glykopeptide und Lipide,
- CD 20 der B-Lymphozyten, Epithel spezifisches Antigen (ESA), Antihuman Episialin (EMA), Farnesyl Transferase, Cyclin dependant Kinase, E1-A-Protein ATM, MLH 1, XP-A, XP-B, MGMT, BLM, NF1 PTH, p27, RB und Caspase Aktivatoren,
- humane reverse Transskriptase und CD 20 der B-Lymphozyten,
- CD 5 und CD 72 der B-Lymphozyten,
- humane reverse Transskriptase und CD 5 und CD 72 B-Lymphozyten, Epithel spezifisches Antigen (ESA), Antihuman Episialin (EMA), Farnesyl Transferase, Cyclin dependant Kinase, E1-A-Protein ATM, MLH 1, XP-A, XP-B, MGMT, BLM, NF1 PTH, p27, RB und Caspase Aktivatoren,
- CD 2, CD 3 und CD 20 von B-Lymphozyten,
- die reverse Transskriptase vom human-Typ sowie gegen CD 2, CD 3, CD 5, CD 7 und T-Lymphozyten,
- CD 2, CD 3, CD 5, CD 7 vom monoklonalen Typ gegen T-Lymphozyten sowie Epithel spezifisches Antigen (ESA), Anti-human Episialin (EMA), Farnesyl Transferase, Cyclin dependant Kinase, E1-A-Protein ATM, MLH 1, XP-A, XP-B, MGMT, BLM, NF1 PTH, p28, RB und Caspase Aktivatoren,
- die reverse Transskriptase vom humanen Typ sowie CD 2, CD 3, CD 5, CD 7 der T-Lynphozyten sowie gegen Epithel spezifisches Antigen (ESA),
- CD 13, CD 14, CD 15, CD 33, CD 34 der Myeloid-LeukämieZellen,
- CD 13, CD 14, CD 15, CD 33, CD 34 der Myeloid-LeukämieZellen sowie gegen Epithel spezifisches Antigen (ESA), Antihuman Episialin (EMA), Farnesyl Transferase, Cyclin dependant Kinase, E1-A-Protein ATM, MLH 1, XP-A, XP-B, MGMT, BLM, NF1 PTH, p27, RB und Caspase Aktivatoren,
- humane reverse Transskriptase sowie HLA-DR, HLA B 3503 und CD 34 Antikörper,
- humane reverse Transskriptase, CD 5 der T-Lymphozyten sowie gegen Epithel spezifisches Antigen (ESA) Antikörper,
- humane reverse Transskriptase sowie gegen Promyleozyten vom Typ PG-M3 (Moab) und den Retinoidsäure-Rezeptor alpha,
- humane reverse Transskriptase sowie gegen Makrophagen vom Typ (MAb) sowie gegen Epithel spezifisches Antigen (ESA), Antihuman Episialin (EMA), Farnesyl Transferase, Cyclin dependant Kinase, E1-A-Protein ATM, MLH 1, XP-A, XP-B, MGMT, BLM, NF1 PTH, p27, RB und Caspase Aktivatoren,
- die reverse Transskriptase, das Protein A, Conductin-Protein, SDF-1-Protein, 6-C-Kine-Protein, MIP-3β-Protein und VCAM,
- die reverse Transskriptase, das P-Glykoprotein (MDR) und P-Glykoprotein (MDR) -Fragmente,
- die reverse Transskriptase, vaskulären endothelialen Wachstumstaktor (VEGF) und (VEFG-Fragmente), (VEGF-Rezeptor Antagonisten),
- die reverse Transskriptase, gegen Epithel spezifisches Antigen (ESA), gegen die Matrixmetalloproteinase-2 (MMP-2) und/oder gegen den Aktivator der Matrixmetalloproteinase-1 (MT1-MMP),
- die bei der Gentherapie mit Oligodeoxynukleotiden im Rahmen Antisense Technik auftretenden Antikörper oder gegen die bei der Gentherapie der Genersatztherapie, der Gensequenzänderungstherapie oder gegen die bei der Virus Vektor Gentherapie oder gegen die bei der Virus Vektor Augmentationstherapie oder gegen die bei der Artifiziellen Chromosomen-Therapie oder gegen die bei der DNS-Plasmid-Therapie oder die bei der Immunogen-Therapie, gegen die bei der Tumor-Supressor-Onkogene-Apoptosis-Kombinationstherapie, gegen die bei der Gentherapie mit Rekombinanten-Material entstehenden Antikörper,
- die reverse Transskriptase, das Protein HER-2/neu, gegen Antihuman Episialin (EMA) und gegen VCAM,
- die reverse Transskriptase, das Protein HER-2/neu, MYC, ERB B1-3, RAS, MSH2, p53 und VCAM,
- die reverse Transskriptase, gegen die durch das Krebswachstum mutierten Proteine HER-2/neu, MYC, ERB B1-3, RAS, MSH2, p53 und/oder VCAM,
- die reverse Transskriptase, die durch das Krebswachstum mutierte Proteine, RAS, MSH2, p53 sowie VCAM,
- das Endothelin (VEGF), (VEGF-Fragmente), (VEGF-Rezeptor Antagonisten), Matrix-Proteine, VCAM, Catenin (alpha, beta), neurale Zelladhäsionsproteine und Cadherine,
- das Endothelin (VEGF), (VEGF-Fragmente), (VEGF-Rezeptor Antagonisten), VCAM und HLA und humane Leukozyten,
- das Endothelin (VEGF), (VEGF-Fragmente), (VEGF-Rezeptor Antagonisten), (VEGF), (VEGF-Fragmente), (VEGF-Rezeptor Antagonisten), VCAM und gegen HLA und/oder gegen humane Leukozyten und/oder T-Lymphozyten,
- das VCAM-Protein und/oder gegen Rezeptor für Glykat-Endprodukte und/oder gegen Hemozoin,
- das lösliche Plasmodium falciparum Antigen, das VCAM-Protein, gegen Rezeptor für Glykat-Endprodukte und Hemozoin,
- das lösliche Plasmodium falciparum Antigen, das VCAM- und ICAM-Protein, gegen den Rezeptor für Glykat-Endprodukte und gegen Hemozoin,
- das Plasmodium falciparum Antigen, die Glykosyl-phosphatidylinositol-Produkte (GPI) aus Plasmodium falciparum, aus Trypanosoma brucei oder aus Leishmania mexicana, gegen das VCAM- und ICAM-Protein, gegen den Rezeptor für Glykat-Endprodukte und Hemozoin,
- das lösliche Plasmodium falciparum, aus Trypanosoma brucei oder aus Leishmania mexicana, gegen das VCAM- und gegen das ICAM-Protein, gegen den Rezeptor für Glykat-Endprodukte, gegen Hemozoin und Lipopolysaccharide,
- Hemozoin und den Endothelrezeptor vom Typ PECAM-1/CD31,
- das Mycobakterium leprae, das Mitsuda Antigen und das Dharmendra Antigen,
- das Mycobakterium leprae, das Mitsuda Antigen, das Dharmendra Antigen sowie gegen das VCAM- und ICAM-Protein,
- das Protein A und das Protein p59 fyn der Kinase,
- die reverse humane Transskriptase, gegen das Protein A, das Protein p59 fyn der Kinase und das Aktivator-Protein-1 (AP-1),
- die reverse humane Transskriptase, das Protein A, gegen das Protein p59 fyn der Kinase, das Aktivator-Protein-1 (AP-1) und gegen das Serum Amyloid-A-Protein (SAA),
- die reverse humane Transskriptase, das Protein A, gegen das Protein p59 fyn der Kinase, das Aktivator-Protein-1 (AP-1), das Serum Amyloid-A-Protein (SAA), Plasminogen-Aktivator-Inhibitor-Typ-1-Antigen (PAI-1) sowie gegen Farnesyl Transferase, Cyclin dependant Kinase und Caspase Aktivatoren,
- die reverse humane Transskriptase, Protein-1 (AP-1), gegen das Serum beta-Amyloid-Protein, beta-Amyloid-Protein-Fragmente, beta-Amyloid-Präkursor-Protein (beta APP) und gegen das Oncostatin-M-Protein (OSM),
- die reverse humane Transskriptase, gegen das mutierte p53-Protein und gegen das Ki.67-Antigen, sowie gegen Farnesyl Transferase, Cyclin dependant Kinase und Caspase Aktivatoren,
- die reverse humane Transskriptase, gegen das mutierte p53-Protein und gegen die, die bei Anwendung von Gen-transfizierten Cholesterol-Liposomen gegen Tumore entstehen,
- die reverse Transskriptase, gegen das mutierte p53-Protein und gegen den vakulären endothelialen Gefäßwachstumsfaktor (VEGF), (VEGF-Fragmente), (VEGF-Rezeptor Antagonisten), sowie gegen Conductin und P-Selectin,
- die reverse humane Transskriptase, gegen das beta-2-Mikro-globulin (B2-MG) und gegen den vakulären endothelialen Gefäßwachstumstaktor (VEGF), (VEGF-Fragmente), (VEGF-Rezeptor Antagonisten), sowie gegen Farnesyl Transferase, Cyclin dependant Kinase und Caspase Aktivatoren,
- das beta-2-Mikroglobulin (B2-MG), gegen den vakulären endothelialen Wachstumstaktor Endothelin (VEGF), (VEGF-Fragmente), (VEGF-Rezeptor Antagonisten), sowie gegen Farnesyl Transferase, Cyclin dependant Kinase und Caspase Aktivatoren vom monoklonalen Typ,
- den Plasma Endothelin (VEGF), (VEGF-Fragmente), (VEGF-Rezeptor Antagonisten) (VEGF), gegen das Oncostatin Protein und gegen Interleukin 6,
- die reverse humane Transskriptase und gegen das Rezeptor Antigen CD 4,
- reverse humane Transskriptase, gegen das Rezeptor Antigen CD 4 vom monoklonalen Typ und HIV-Protease-Inhibitoren,
- reverse humane Transskriptase, das Rezeptor Antigen CD 4 und gegen das p24 Antigen,
- das Mycobakterium tuberkulosis und das BCG-Antigen,
- Mycobakterium tuberkulosis, das BCG-Antigen, Endothelin (VEGF), (VEGF-Fragmente), (VEGF-Rezeptor Antagonisten), VCAM, Telomerase sowie gegen Farnesyl Transterase, Cyclin dependant Kinase und Caspase Aktivatoren,
- das Endothelin (VEGF), (VEGF-Fragmente), (VEGF-Rezeptor Antagonisten)-Protein, VCAM, gegen HLA, humane Leukozyten, T-Lymphozyten und gegen das P-Selectin,
- HLA, humane Leukozyten, T-Lymphozyten, extrazelluläre Matrix und gegen das P-Selectin,
- das eosinophile kationische Protein (ECP) vom monoklonalen Typ,
- die reverse humane Transskriptase, das mutierte p53-Protein und das Eppstein-Barr-Virus und gegen das E1A-Virusprotein,
- das Endothelin (VEGF), (VEGF-Fragmente), (VEGF-Rezeptor Antagonisten), VCAM und das HLA-B-7-Residuum 75-84 der alpha-Domäne, gegen humane Leukozyten, T-Lymphozyten und gegen das P-Selectin,
- das Endothelin (VEGF), (VEGF-Fragmente), (VEGF-Rezeptor Antagonisten), VCAM und gegen das HLA-B-7-Residuum 75-84 der alpha-Domäne,
- das Endothelin (VEGF), (VEGF-Fragmente), (VEGF-Rezeptor Antagonisten), VCAM und gegen A-beta-2-M-Amyloid,
- das Endothelin (VEGF), (VEGF-Fragmente), (VEGF-Rezeptor Antagonisten), VCAM und gegen A-beta-2-M-Amyloid, Conductin-Protein, SDF-1-Protein, 6-C-Kine-Protein und MIP-3β-Protein,
- das A-beta-2-M-Amyloid-Selectin (P,L), CD 994, NKG 2A BCL2, Endothelin (VEGF) und (VEGF-Fragmente), (VEGF-Rezeptor Antagonisten),
- Gallensäuren, Ammoniak und Bilirubin,
- CD 3, LAF, A-beta-2-M-Amyloid-Selectin (P,L), CD 994, NKG 2A BCL2, Endothelin (VEGF), (VEGF-Fragmente) und (VEGF-Rezeptor Antagonisten),
- CD 994, NKG 2A, Endothelin (VEGF), (VEGF-Fragmente) und (VEGF-Rezeptor Antagonisten),
- CD 3 und LFA-1-alpha,
- MDR 1-Protein vom Typ MRK 16 und C 219, Endothelin (VEGF), (VEGF-Fragmente), (VEGF-Rezeptor Antagonisten), Protein A und P-Selectin,
- MDR1-Protein vom Typ MRK 16 und C 219, Endoethlin (VEGF), (VEGF-Fragmente), (VEGF-Rezeptor Antagonisten), (VEGF), (VEGF-Fragmente) und (VEGF-Rezeptor Antagonisten) -Protein,
- die Antikörper des Fusionsproteins aus Fab Teil (aus dem monoklonalen Antikörper C 215) und dem -SEA (Staphylokocken Enterotoxin Antigen), Endothelin (VEGF), (VEGF-Fragmente) und (VEGF-Rezeptor Antagonisten),
- Epidermal Growth Faktor (EGFR), Endothelin (VEGF), (VEFG-Fragmente), (VEGF-Rezeptor Antagonisten), Farnesyl Transferase, Cyclin dependant Kinase (CDK), gegen Caspase Aktivatoren und Selectin (P,L),
- Epidermal Growth Faktor (EGFR), Endothelin (VEGF), (VEGF-Fragmente), (VEGF-Rezeptor Antagonisten) (VEGF), (VEGF-Fragmente), (VEGF-Rezeptor Antagonisten) -Protein, Selectin (P,L), gegen Interleukin 6 oder Interleukin Gen exprimierende Konstrukte,
- Epidermal Growth Faktor (EGFR), Endothelin (VEGF), (VEGF-Fragmente), (VEGF-Rezeptor Antagonisten) (VEGF), (VEGF-Fragmente), (VEGF-Rezeptor Antagonisten) -Protein, Selectin (P,L) und OX 40,
- HLA B 3503,
- Interleukin-8,
- Epidermal Growth Faktor (EGFR), (Endothelin (VEGF), (VEGF-Fragmente), (VEGF-Rezeptor Antagonisten), Selectin (P,L) und Interleukin-1,
- Epidermal Growth Faktor (EGFR), Endothelin (VEGF), (VEGF-Fragmente), (VEGF-Rezeptor Antagonisten), Selectin (P,L) und Interleukin-2-Rezeptor,
- HLA-DR 1,2,4, Oligodendrozyten Glykoprotein (MOG), gegen den Inhibitor des Nervenwachstums vom Typ NI 35/250 sowie gegen neurotrophische Stimulatoren und gegen Semaphorin Epidermal Growth Faktor (EGFR), Endothelin (VEGF), (VEGF-Fragmente), (VEGF-Rezeptor Antagonisten), Selectin (P,L) und Squamöses Zell-Karzinom-Antigen (SCC),
- Epidermal Growth Faktor (EGFR), Endothelin (VEGF), (VEGF-Fragmente), (VEGF-Rezeptor Antagonisten), Selectin (P,L) und Matrix-Protein--Tenascin-C (TN-C),
- Epidermal Growth Faktor (EGFR), Endothelin (VEGF), (VEGF-Fragmente), (VEGF-Rezeptor Antagonisten), Selectin (P,L) und gegen Sialyl Lewis x,
- Epidermal Growth Faktor (EGFR), Endothelin (VEGF), (VEGF-Fragmente), (VEGF-Rezeptor Antagonisten), Selectin (P,L) und gegen IGM-Zytostatica Kombinationen,
- Protein A, gegen Human HLA class 1 Antigen, gegen Anti-Thrombopeotin, Lipopolysaccharici und gegen Van Glykoprotein,
- Epidermal Growth Faktor (EGFR, Endothelin (VEGF), (VEGF-Fragmente), (VEGF-Rezeptor Antagonisten), Selectin (P,L) und NF-kappa-B-Gen-exprimierende Konstrukte,
- Epidermal Growth Faktor (EGFR), Endothelin (VEGF), (VEGF-Fragmente), (VEGF-Rezeptor Antagonisten), Selectin (P,L) und Endothel-aktivierendes Polypeptid II (EMAP II),
- Epidermal Growth Faktor (EGFR), Endothelin (VEGF), (VEGF-Fragmente), (VEGF-Rezeptor Antagonisten), Selectin (P,L) und das Antiprotein gegen den Endothel aktivierendes Polypeptid II (EMAP II),
- Epidermal Growth Faktor (EGFR), Endothelin (VEGF), (VEGF-Fragmente), (VEGF-Rezeptor Antagonisten), Selectin (P,L), Anti CD3 und Glykoprotein EGP2 als bispezifischem Antikörper,
- Epidermal Growth Faktor (EGFR), Endothelin (VEGF), (VEGF-Fragmente), (VEGF-Rezeptor Antagonisten), Selectin (P,L), Anti-Makrophagen-Migration-inhibitorischem Faktor (Anti-MIF), Protein A, Conductin-Protein, SDF-1-Protein, 6-C-Kine-Protein und MIP-3β,
- Epidermal Growth Faktor (EGFR), Endothelin (VEGF), (VEGF-Fragmente), (VEGF-Rezeptor Antagonisten), Selectin (P,L) und Anti Leukämie-inhibitorischen Faktor (Anti LIF),
- Epidermal Growth Faktor (EGFR), Endothelin (VEGF), (VEGF-Fragmente), (VEGF-Rezeptor Antagonisten), Selectin (P, L) wirken und Anti-N-ramp-Gen-Konstrukt,
- Epidermal Growth Faktor (EGFR), Endothelin (VEGF), (VEGF-Fragmente), (VEGF-Rezeptor Antagonisten), Selectin (P,L), Substanz P (SP) und Substanz-SP-Fragmente,
- Epidermal Growth Faktor (EGFR), Endothelin (VEGF), (VEGF-Fragmente), (VEGF-Rezeptor Antagonisten), Selectin (P,L) und Interleukin-1-Rezeptor,
- Epidermal Growth Faktor (EGFR), Endothelin (VEGF), (VEGF-Fragmente), (VEGF-Rezeptor Antagonisten), Selectin (P,L), Interleukin 2 und Interleukin 4,
- Epidermal Growth Faktor (EGFR), Endothelin (VEGF), (VEGF-Fragmente), (VEGF-Rezeptor Antagonisten), Selectin (P,L), Substanz P (SP) und vasoaktive intestinale Peptide (VIP), Protein A, Conductin-Protein, SDF-1-Protein, 6-C-Kine-Protein, MIP-3β,
- Epidermal Growth Faktor (EGFR), Selectin (P,L), (VEFG), (VEFG-Fragnente), (VEFG-Rezeptor Antagonisten), Farnesyl Transferase, Cyclin dependant Kinase und Proleration,
- die reverse Transskriptase, gegen die durch das Krebswachstum mutierten Proteine HER-2-neu, MYC, ERE 81-3, ERB B2, RAS, 2, p53, gegen VCAM, gegen Epidermal Growth Faktor (EGFR), Selectin (P, L), (VEGF), (VEGF-Fragmente und (VEGF-Rezeptor Antagonisten),
- P-Glykoprotein und P-Glykoprotein-Fragmente, gegen Endorphine, Enkephaline, Melanozyten stimulierende Hormone, Opiod, Substanz P und gegen deren Peptide und Rezeptorantagonisten und Antagonisten,
- CD 4, gegen den MHC II (Major Histokompatibilitätskomplex)-Peptid-Komplex und gegen LFA-1 (Leukozyten-Funktion assoziiertes Antigen),
- das lösliche Plasmodium falciparum Antigen, gegen das VCAM-Protein und/oder gegen Rezeptor für Glykat-Endprodukte und/oder gegen das cp 26 und cp 29, Circumsporozoit Proteins des Plasmodium falciparum und/oder gegen Hemozoin,
- Epidermal Growth Faktor (EGFR), Endothelin (VEGF), (VEGF-Fragmente), (VEGF-Rezeptor Antagonisten), Selectin (P,L), Endothel aktivierendes Polypeptid II (EMAP II), Protein A, Conductin-Protein, SDF-1-Protein, 6-C-Kine-Protein und MIP-3β,
- CD 4 und gegen MHC II (Major Hisotkompatibilitätskomplex)-Peptid-Komplex,
- CD 8 und gegen MHC I (Major Histokompatibilitätskomplex)-Peptid-Komplex,
- Epidermal Growth Faktor (EGFR), Selectin (P,L), (VEGF), (VEGF-Fragmente), (VEGF-Rezeptor Antagonisten) und Neuropilin als spezifischen Inhibitor der Gefäßneublidung und endothelialen Proliferation,
- HLA-DR 1,2,4 und gegen Oligodendrozyten Glykoprotein und gegen Semaphorin als Antikörper vom monoklonalen Typ (MOG),
- HLA-DR 1,2,4, gegen Oligodendrozyten Glykoprotein (MOG), gegen das Epstein-Barr-Virus (EBV) und ggen Semaphorin Protein,
- die reverse Transskriptase, gegen die durch das Krebswachstum mutierten Proteine HER-2-neu, MYC, ERB B1-3, RAS, MSH2, p 53, VCAM, Epidermal Growth Faktor (EGFR), Selectin (P,L), Sphingosine-1-Phosphate (SPP), (VEGF), (VEFG-Fragmente) und (VEFG-Rezeptor Antagonisten) vom monoklonalen Typ,
- die reverse Transskriptase, gegen die das Krebswachstum blockierenden Faktoren, gegen HER-2-neu, MYC, ERB B1-3, RAS, MSH2, p53, VCAM, Epidermal Growth Faktor (EGFR), Selectin (P,L), (VEFG), (VEFG-Fragmente), (VEFG-Rezeptor Antagonisten), Farnesyl Transferase und Cyclin dependant Kinase (CDK) vom monoklonalen Typ,
- die reverse Transskriptase, gegen die durch das Krebswachstum mutierten Proteine HER-2-neu, BRCA 1, BRCA 2, RAS, MSH2, p 53, Farnesyl Transferase, Cyclin dependant Kinase und/oder VCAM, Epidermal Growth Faktor (EGFR), Selectin (P,L), (VEGF), (VEFG-Fragmente) und (VEFG-Rezeptor Antagonisten vom monoklonalen Typ,
- Epidermal Growth Faktor (EGFR), Endothelin (VEGF), (VEGF-Fragmente), (VEGF-Rezeptor Antagonisten), Selectin (P,L), Endothel aktivierendes Polypeptid II (EMAP II), Conductin-Protein, SDF-1-Protein und 6-C-Kine-Protein,
- die reverse Transskriptase, gegen die durch das Krebswachstum mutierten Proteine HER-2-neu, BRCA 1, BRCA 2, RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor-Antagonisten) und E1-A-Protein vom monoklonalen Typ,
- CD 4, gegen CD 8, gegen den MHC II (Major Histokompatibilitätskomplex) -Peptid-Komplex, MHC-I-Peptidkomplex und gegen Peptidyl Caspase-Inhibitoren,
- die reverse Transskriptase, gegen die durch das Krebswachstum mutierten Proteine HER-2-neu, BRCA 1, BRCA 2, RAS, MSH 2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein und gegen Caspase Aktivatoren vom monoklonalen Typ,
- HLA-DR 1,2,4- gegen Oligodendrozyten Glykoprotein (MOG), gegen das Epstein-Barr-Virus (EBV), Polypeptid Neurotoxin und gegen Sempaphorin Protein als Antikörper vom monoklonalen Typ,
- HLA-DR 1,2,4- gegen Oligodendrozyten Glykoprotein (MOG), gegen das Epstein-Barr-Virus (EBV), gegen Mikrotubulin assoziierte Proteine (MAPe), (MAPs, Tau-Proteine) und gegen Sempaphorin Protein als Antikörper vom monoklonalen Typ,
- CD 4, gegen den MHC II (Major Histokompatibilitätskomplex) -Peptid-Komplex, gegen LFA-1 (Leukozyten-Funktion assoziiertes Antigen) und gegen HIV-Proteinase-Inhibitor,
- CD4, gegen den MHC II (Major Histokompatibilitätskomplex)-Peptid-Komplex, gegen LFA-1 (Leukozyten-Funktion assoziiertes Antigen), gegen ZIV-Proteinase, gegen Integrin und gegen Tristetraprolin,
- CD 4, gegen den MHC II (Major Histokompatibilitätskomplex) -Peptid-Komplex, gegen LFA-1 (Leukozyten-Funktion assoziiertes Antigen), gegen HIV-Proteinase-Inhibitor, gegen Integrin und Viruscapside, Glykopeptide,
- die reverse Transskriptase, gegen die durch das Krebswachstum mutierten Proteine HER-2-neu, BRCA 1, BRCA 2, RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein ATM, MLH 1, XP-A, XP-B, MGMT, BLM, NF1 PTH, p27, RB und gegen Caspase Aktivatoren vom monoklonalen Typ,
- Kreatinin, Harnsäure, Harnstoff und Lipide und gegen beta-Mikroglobulin und Amyloid,
- Cholera Toxin, Pseudomonas Exotoxin A, Staphylococcus alpha Toxin und Staphylococcus Enterotoxin A und B,
- die reverse Transskriptase, gegen die durch das Krebswachstum mutierten Proteine HER-2-neu, Epithel spezifisches Antigen (ESA), RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein ATM, MLH 1, XP-A, XP-B, MGMT, BLM, NF1 PTH, p27, RB und gegen Caspase Aktivatoren vom monoklonalen Typ,
- die reverse Transskriptase, gegen die durch das Krebswachstum mutierten Proteine HER-2-neu, Epithel spezifisches Antigen (ESA), Antihuman Episialin (EMA), RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein ATM, MLH 1, XP-A, XP-B, MGMT, BLM, NF1 PTH, p27, RB und gegen Caspase Aktivatoren vom monoklonalen Typ,
- die reverse Transskriptase, gegen Proteine HER-2-neu, Epithel spezifisches Antigen (ESA), Antihuman Episialin (EMA), RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein ATM, MLH 1, XP-A, XP-B, MGMT, BLM, NF1 PHT, p27, RB und gegen Caspase Aktivatoren, gegen die Matrixmetalloproteinase-2- (MMP-2) und/oder gegen den Aktivator der Matrixmetaloproteinase-1 (MT1-MMP),
- HLA-DR 1,2,4, gegen Oligodendrozyten Glykoprotein (MOG), gegen den Inhibitor des Nervenwachstums vom Typ NI 35/250 sowie gegen neurotrophische Stimulatoren und gegen Semaphorin
enthalten.

Die erfindungsgemäßen Trägermaterialien können auch Mischungen der oben angegebenen Antikörper in der Beschichtung enthalten, solange dies therapeutisch sinnvoll ist.

Die Gewinnung von Antikörpern und deren Einsatz erfolgt wie unten beschrieben:

Die unterschiedlichen Antikörper für die einzelnen Krankheitsbilder werden als polyglonale Antikörper von Tieren gewonnen. Besonders geeignet sind hier folgende Tierspezies: Schwein, Schaf, Ziege, Kaninchen, Ratten und Mäuse.

Zur Bildung der Antikörper werden den Tieren die krankmachenden Verbindungen, z.B. Amyloid, TNF, zusammen mit Freudschen Adjuvans in den Nacken, die Fußsohlen, sowie an vier unterschiedlichen Muskelarealen injiziert.

Nach zwei Wochen wird diese Behandlung wiederholt (Booster), so daß dieser Vorgang insgesamt jeweils 5 mal durchgeführt wird. Durch die Injektionen wird die Bildung der spezifischen Antikörper gewährleistet.

10 Tage nach der letzten Antigenapplikation wird den Tieren Blut, bzw. Plasma entnommen, in dem sich die gebildeten Antikörper befinden. Durch Separationsverfahren werden diese Antikörper konzentriert und über ausgewählte Filtrationsmethoden gereinigt.

Diese Antikörper werden auf das Trägermaterial gebunden, wobei eine kovalente Bindung bevorzugt wird. Das Trägermaterial besteht beispielsweise aus Sepharose wie z.B. vom Typ IV CLB, feinstgranulierten Kunststoffen aus PUR, Silikon, Glasverbindungen mit vorbereiteten Bindungsgruppen wie -SH zur Bindung der Antikörper.

Die an das Trägermaterial gebundenen Antikörper werden in ein Behältnis, das über eine Zu- und Ableitung verfügt, gebracht. Das Patientenblut wird Über eine Plasmaphereseanlage aufgetrennt und das Plasma über die gebundenen Antikörper geleitet. Dabei werden die Krankheit auslösenden Moleküle an die spezifischen Antikörper im Sinne einer Antikörper-Antigenverbindung aus dem Plasma entfernt. Die auf den radialen Oberflächen kovalent gebundenen Antikörper werden als gesamtes Immunglobulin, bestehend aus F(ab)₂-Teilen und dem Fc-Stück aufgebracht. Durch die Beibehaltung des Fc-Teils ist eine dichtere Packung der Immunglobuline möglich, da der lineare Verlauf des Fc-Teils den Radius und somit die Fläche für die F(ab)₂-Teile vergrößert.

Der potentielle Nachteil durch die Belassung des Fc-Stücks liegt vor allem in der Komplementaktivierung. Dieser Risikofaktor wird dahingehend eliminiert, indem man die Ca²⁺-Ionen im Plasma durch Zugabe einer angesäuerten Lösung in das Plasma bindet. Die Komplementaktivierung benötigt für den Ablauf zu Beginn Ca²⁺-Ionen als Startermoleküle. Da diese durch die Vorbehandlung nicht mehr vorliegen, ist eine Komplementaktivierung ausgeschaltet.

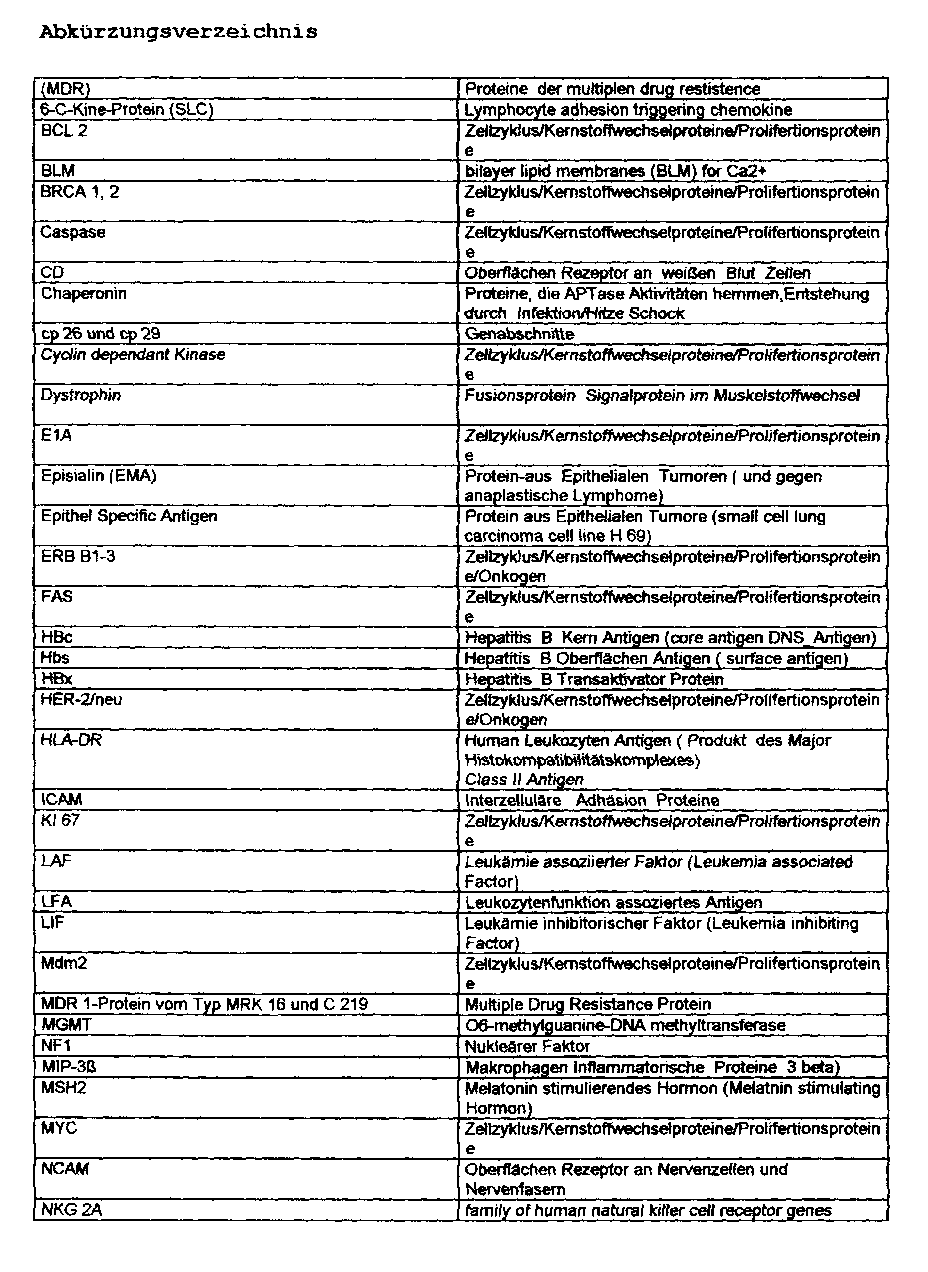

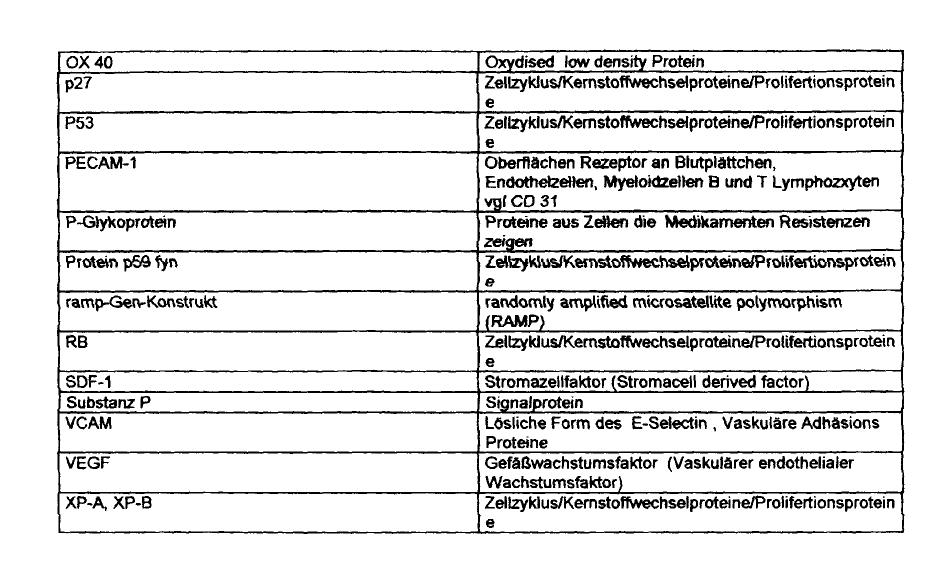

## Patentansprüche

1. Trägermaterial, umfassend eine Beschichtung aus den jeweiligen Antikörpern gegen TNF und/oder gegen Anti-TNF und/oder gegen Fragmente von TNF und/oder gegen Fragmente von Anti-TNF oder gegen TNF- Transportproteine , wobei die Beschichtung weitere Antikörper umfaßt, die gegen spezifische Krankheitserreger wirksam sind.

2. Trägermaterial nach Anspruch 1, dadurch gekennzeichnet, daß die weiteren Antikörper gegen ein Bakterien/Virusgemisch wirken, bestehend aus Staphylococcus aureus, Streptococcus mitis, Haemophilus influenza, Diploccocus pneumoniae, Klebsiella pneumoniae und ozeanae, Staphylococcus pyogenses und viridans, Newisseria cateraus, E. Coli, β-hämolisierende Streptococcus, Hepatitis A Antigen und Hbs Antigen oder die weiteren Antikörper monoklonale und/oder polyklonale Antikörper gegen die genannten Antigene sind.

3. Trägermaterial nach Anspruch 1 oder 2, dadurch gekennzeichnet, daß die weiteren Antikörper gegen ein Bakterien/Virus-Gemisch wirken, bestehend aus Staphylococcus aureus, Streptococcus mitis, Haemophilus influenza, Diplococcus pneumoniae, Klebsiella pneumoniae und ozeanae, Staphylococcus pyogenses und viridans, Neisseria cateralis, E. Coli und β-hämolisierende Strepococcus oder die weiteren Antikörper monoklonale und/oder polykionale Antikörper gegen die genannten Antigene sind.

4. Trägermaterial nach einem oder mehreren der Ansprüche 1 bis 3, dadurch gekennzeichnet, daß die weiteren Antikörper gegen die Ribosomen der Bakterien wirken, bestehend aus Staphylococcus aureus, Streptococcus mitis, Haemophilus influenza, Diplococcus pneumoniae, Klebsiella pneumoniae und ozeanae, Staphylococcus pyogenses und viridans, Neisseria cateralis, E. Coli und β-hämolisierende Streptococcus, sowie Antikörper gegen Hepatitis A Antigen, Hbs Antigen sind, oder die weiteren Antikörper monoklonale und/oder polyklonale Antikörper gegen die genannten Antigene sind.

5. Trägermaterial nach einem oder mehreren der Ansprüche 1 bis 4, dadurch gekennzeichnet, daß die weiteren Antikörper gegen die Ribosomen der Bakterien wirken, bestehend aus Styphylococcus aureus, Streptococcus mitis, Haemophilus influenza, Diploccocus pneumoniae, Klebsiella pneumoniae und ozeanae, Staphylococcus pyogenses und viridans, Neisseria cateralis, E. Coli und β-härnolisierende Streptococcus, oder die weiteren Antikörper monoklonale und/oder polyklonale Antikörper gegen die genannten Antigene sind.

6. Trägermaterial nach einem oder mehreren der Ansprüche 1 bis 5, dadurch gekennzeichnet, daß die weiteren Antikörper gegen β-hämolysierende Streptokokken A und gegen Virus Capside wirken, oder die weiteren Antikörper monoklonale und/oder polykionale Antikörper gegen die genannten Antigene sind.

7. Trägermaterial nach einem oder mehreren der Ansprüche 1 bis 6, dadurch gekennzeichnet, daß die weiteren Antikörper gegen Polysaccharide von Streptococcus pneumoniae und gegen Virus Capside, Chiamydien und Myköpiasmen wirken, oder die weiteren Antikörper monoklonale und/oder polyklonale Antikörper gegen die genannten Antigene sind.

8. Trägermaterial nach einem oder mehreren der Ansprüche 1 bis 7, dadurch gekennzeichnet, daß die weiteren Antikörper gegen Anti-I-cold Agglutinin, gegen Polysaccharide von Streptococcus pneumoniae und gegen Virus Capside, Chlamydien und Mykóplasmen wirken, oder die weiteren Antikörper monoklonale und/oder polyklonale Antikörper gegen die genannten Antigene sind.

9. Trägermaterial nach einem oder mehreren der Ansprüche 1 bis 8, dadurch gekennzeichnet, daß die weiteren Antikörper gegen Polysaccharide von Streptococcus pneumoniae und Antikörper gegen Influenzaviren, Influenzaviren, Influenza Hänagglutinin Peptide, Hämagglutinin Peptide wirken, oder die weiteren Antikörper monoklonale und/oder polyklonale Antikörper gegen die genannten Antigene sind.

10. Trägerinaterial nach einem oder mehreren der Ansprüche 1 bis 9, dadurch gekennzeichnet, daß die weiteren Antikörper gegen Polysaccharide von Streptococcus pneumoniae und Antikörper gegen Influenzaviren, Influenza Hämagglutinin Peptide sowie gegen Anti-I-cold Agglutinin wirken, oder die weiteren Antikörper monoklonale und/oder polyklonale Antikörper gegen die genannten Antigene sind.

11. Trägermaterial nach einem oder mehreren der Ansprüche 1 bis 10, dadurch gekennzeichnet, daß die weiteren Antikörper gegen ein Bakterien/Virus-Gemisch wirken, bestehend aus Staphyloccocus aureus, Streptococcus mitis, Haemophilus influenza, Diplococcus pneumoniae, Klebsiella pneumoniae und ozeanae, Staphylococcus pyogenses und viridans, Neisseria cateralis, E. Coli, β-hämolisierende Streptococcus A, Hepatitis A Antigen, Hbs Antigen, Influenzaviren und Influenza Hämagglutinin Peptide, sowie Antikörper gegen Protein A wirken, oder die weiteren Antikörper monoklonale und/oder polyklonale Antikörper gegen die genannten Antigene sind.

12. Trägermaterial nach einem oder mehreren der Ansprüche 1 bis 11, dadurch gekennzeichnet, daß die Antikörper gegen die reverse Transskriptase, gegen Hepatitis A Antigen und Virus Capside wirken, oder die weiteren Antikörper monoklonale und/oder polyklonale Antikörper gegen die genannten Antigene sind.

13. Trägermaterial nach einem oder mehreren der Ansprüche 1 bis 12, dadurch gekennzeichnet, daß die weiteren Antikörper gegen die reverse Transskriptase, gegen Hepatitis B Antigen, (Hbs) ,gegen Hepatitis B Kernmaterialien (DNS) (HBc), und Virus Capside wirken, oder die weiteren Antikörper monoklonale und/oder polyklonale Antikörper gegen die genannten Antigene sind.

14. Trägermaterial nach einem oder mehreren der Ansprüche 1 bis 13, dadurch gekennzeichnet, daß die weiteren Antikörper gegen die reverse Transskriptase, gegen Hepatitis B Antigen, (Hbs) und gegen das Transaktivator Hbx Protein wirken, oder die weiteren Antikörper monoklonale und/oder polyklonale Antikörper gegen die genannten Antigene sind.

15. Trägermaterial nach einem oder mehreren der Ansprüche 1 bis 14, dadurch gekennzeichnet, daß die weiteren Antikörper gegen die reverse Transskriptase und gegen Hepatitis C Virus Genom (Nukleocapsid Protein) wirken, oder die weiteren Antikörper monoklonale und/oder polyklonale Antikörper gegen die genannten Antigene sind.

16. Trägermaterial nach einem oder mehreren der Ansprüche 1 bis 15, dadurch gekennzeichnet, daß die weiteren Antikörper gegen die reverse Transskriptase, gegen Hepatitis C Antigen, Protein A, Herpes, Influenzaviren und Influenza Hämagglutinin Peptide wirken, oder die weiteren Antikörper monoklonale und/oder polyklonale Antikörper gegen die genannten Antigene sind.

17. Trägermaterial nach einem oder mehreren der Ansprüche 1 bis 16, dadurch gekennzeichnet, daß die weiteren Antikörper gegen β-hämolysierende Streptokokken, Endothelin (VEGF), (VEGF-Fragmente), (VEGF-Rezeptor Antagonisten), VCAM, Protein A, Conductin-Protein, SDF-1 Protein, 6-C-Kine-Protein und MIP-3β-Protein wirken, oder die weiteren Antikörper monoklonale und/oder polyklonale Antikörper gegen die genannten Antigene sind.

18. Trägermaterial nach einem oder mehreren der Ansprüche 1 bis 17, dadurch gekennzeichnet, daß die weiteren Antikörper gegen β-hämolysierende Streptokokken, Protein A, P-Glykoprotein und Lipide wirken, oder die weiteren Antikörper monoklonale und/oder polyklonale Antikörper gegen die genannten Antigene sind.

19. Trägermaterial nach einem oder mehreren der Ansprüche 1 bis 18, dadurch gekennzeichnet, daß die weiteren Antikörper gegen HLA-DR 1,2,4, Endothelin (VEGF), (VEGF-Fragmente), (VEGF-Rezeptor Antagonisten), VCAM, Protein A, Conductin-Protein, SDF-1-Protein, 6-C-Kine-Protein und MIP-3β-Protein wirken, oder die weiteren Antikörper monoklonale und/oder polyklonale Antikörper gegen die genannten Antigene sind.

20. Trägermaterial nach einem oder mehreren der Ansprüche 1 bis 19, dadurch gekennzeichnet, daß die weiteren Antikörper gegen HLA-DR 1,2,4, ggegen Virus Capside und gegen Oligodendrozyten Glykoprotein als Antikörper vom monoklonalen Typ (MOG) wirken, oder die weiteren Antikörper monoklonale und/oder polyklonale Antikörper gegen die genannten Antigene sind.

21. Trägermaterial nach einem oder mehreren der Ansprüche 1 bis 20, dadurch gekennzeichnet, daß die weiteren Antikörper gegen HLA-DR 1,2,4, gegen Oligodendrozyten Glykoprotein (MOG) und gegen das Epstein-Barr-Virus (EBV) als Antikörper vom monoklonalen Typ wirken, oder die weiteren Antikörper monoklonale und/oder polyklonale Antikörper gegen die genannten Antigene sind.

22. Trägermaterial nach einen oder mehreren der Ansprüche 1 bis 21, dadurch gekennzeichnet, daß die weiteren Antikörper monoklonale Antikörper gegen erkranktes Organgewebe sind.

23. Trägermaterial nach einem oder mehreren der Ansprüche 1 bis 22, dadurch gekennzeichnet, daß die weiteren Antikörper monoklonale Antikörper gegen erkranktes Schilddrüsengewebe, gegen Dystrophin und AntiDystrophin und gegen Acetylcholin sind.

24. Trägermaterial nach einem oder mehreren der Ansprüche 1 bis 23, dadurch gekennzeichnet, daß die weiteren Antikörper gegen β-hämolysierende Streptokokken, Influenzaviren, Influenza Hämagglutinin Peptide, Lipopolysaccharide und gegen Chaperonin wirken, oder die weiteren Antikörper monoklonale und/oder polyklonale Antikörper gegen die genannten Antigene sind.

25. Trägermaterial nach einem oder mehreren der Ansprüche 1 bis 24, dadurch gekennzeichnet, daß die weiteren Antikörper gegen β-hämolysierende Streptokokken, Van Glykopeptide, Influenzaviren, Influenza Hämagglutinin Peptide, Lipopolysaccharide gegen Matrix-Proteine und beta-2-Mikroglobulin wirken, oder die weiteren Antikörper monoklonale und/oder polyklonale Antikörper gegen die genannten Antigene sind.

26. Trägermaterial nach einem oder mehreren der Ansprüche 1 bis 25, dadurch gekennzeichnet, daß die weiteren Antikörper gegen Kreatinin, Harnsäure, Harnstoff und Lipide wirken, oder die weiteren Antikörper monoklonale und/oder polyklonale Antikörper gegen die genannten Antigene sind.

27. Trägermaterial nach einem oder mehreren der Ansprüche 1 bis 26, dadurch gekennzeichnet, daß die weiteren Antikörper gegen Cocksackie Viren, Virus Capside und Van-Glykopeptide wirken, oder die weiteren Antikörper monoklonale und/oder polyklonale Antikörper gegen die genannten Antigene sind.

28. Trägermaterial nach einem oder mehreren der Ansprüche 1 bis 27, dadurch gekennzeichnet, daß die weiteren Antikörper gegen Herpes, Influenzaviren, Influenza Hämagglutinin Peptide, Cytomegalie Viren, Protein A, Van Glykopeptide und gegen Lipide wirken, oder die weiteren Antikörper monoklonale und/oder polyklonale Antikörper gegen die genannten Antigene sind.

29. Trägermaterial nach einem oder mehreren der Ansprüche 1 bis 28, dadurch gekennzeichnet, daß die weiteren Antikörper monoklonale Antikörper gegen CD 20 der B-Lymphozyten, Epithel spezifisches Antigen (ESA), Antihuman Episialin (EMA), Farnesyl Transferase, Cyclin dependant Kinase, E1-A-Protein ATM, MLH 1, XP-A, XP-B, MGMT, BLM, NF1 PTH, p27, RB und gegen Caspase Aktivatoren wirken, oder die weiteren Antikörper monoklonale und/oder polyklonale Antikörper gegen die genannten Antigene sind.

30. Trägermaterial nach einem oder mehreren der Ansprüche 1 bis 29, dadurch gekennzeichnet, daß die weiteren Antikörper gegen humane reverse Transskriptase und gegen CD 20 der B-Lymphozyten wirken, oder die weiteren Antikörper monoklonale und/oder polyklonale Antikörper gegen die genannten Antigene sind.

31. Trägermaterial nach einem oder mehreren der Ansprüche 1 bis 30, dadurch gekennzeichnet, daß die weiteren Antikörper gegen CD 5 und CD 72 der B-Lymphozyten wirken, oder die weiteren Antikörper monoklonale und/oder polyklonale Antikörper gegen die genannten Antigene sind.

32. Trägermaterial nach einem oder mehreren der Ansprüche 1 bis 31, dadurch gekennzeichnet, daß die weiteren Antikörper gegen humane reverse Transskriptase und gegen CD 5 und CD 72 B-Lymphozyten, Epithel spezifisches Antigen (ESA), Antihuman Episialin (EMA), Farnesyl Transferase, Cyclin dependant Kinase, E1-A-Protein ATM, MLH 1, XP-A, XP-B, MGMT, BLM, NF1 PTH, p27, RB und gegen Caspase Aktivatoren wirken, oder die weiteren Antikörper monoklonale und/oder polyklonale Antikörper gegen die genannten Antigene sind.

33. Trägermaterial nach einem oder mehreren der Ansprüche 1 bis 32, dadurch gekennzeichnet, daß die weiteren Antikörper gegen CD 2, CD 3 und CD 20 von B-Lymphozyten wirken, oder die weiteren Antikörper monoklonale und/oder polyklonale Antikörper gegen die genannten Antigene sind.

34. Trägermaterial nach einem oder mehreren der Ansprüche 1 bis 34, dadurch gekennzeichnet, daß die weiteren Antikörper gegen die reverse Transskriptase vom human-Typ sowie gegen CD 2, CD 3, CD 5, CD 7 und gegen T-Lymphozyten wirken, oder die weiteren Antikörper monoklonale und/oder polyklonale Antikörper gegen die genannten Antigene sind.

35. Trägermaterial nach einem oder mehreren der Ansprüche 1 bis 34, dadurch gekennzeichnet, daß die weiteren Antikörper gegen CD 2, CD 3, CD 5, CD 7 vom monoklonalen Typ gegen T-Lymphozyten sowie gegen Epithel spezifisches Antigen (ESA), Antihuman Episialin (EMA), Farnesyl Transferase, Cyclin dependant Kinase, E1-A-Protein ATM, MLH 1, XP-A, XP-B, MGMT, BLM, NF1 PTH, p28, RB und gegen Caspase Aktivatoren wirken, oder die weiteren Antikörper monoklonale und/oder polyklonale Antikörper gegen die genannten Antigene sind.

36. Trägermaterial nach einem oder mehreren der Ansprüche 1 bis 35, dadurch gekennzeichnet, daß die weiteren Antikörper gegen die reverse Transskriptase vom humanen Typ sowie CD 2, CD 3, CD 5, CD 7 der T-Lymphozyten sowie gegen Epithel spezifisches Antigen (ESA) wirken, oder die weiteren Antikörper monoklonale und/oder polyklonale Antikörper gegen die genannten Antigene sind.

37. Trägermaterial nach einem oder mehreren der Ansprüche 1 bis 36, dadurch gekennzeichnet, daß die weiteren Antikörper gegen CD 13, CD 14, CD 15, CD 33, CD 34 der Myeloid-Leukämie-Zellen wirken, oder die weiteren Antikörper monoklonale und/oder polyklonale Antikörper gegen die genannten Antigene sind.

38. Trägermaterial nach einem oder mehreren der Ansprüche 1 bis 37, dadurch gekennzeichnet, daß die weiteren Antikörper gegen die reverse Transskriptase vom humanen Typ, gegen CD 13, CD 14, CD 15, CD 33, CD 34 der Myeloid-Leukämie-Zellen sowie gegen Epithel spezifisches Antigen (ESA), Antihuman Episialin (EMA), Farnesyl Transferase, Cyclin dependant Kinase, E1-A-Protein ATM, MLH 1, XP-A, XP-B, MGMT, BLM, NF1 PTH, p27, RB und gegen Caspase Aktivatoren wirken, oder die weiteren Antikörper monoklonale und/oder polyklonale Antikörper gegen die genannten Antigene sind.

39. Trägernaterial nach einen oder mehreren der Ansprüche 1 bis 36, dadurch gekennzeichnet, daß die weiteren Antikörper gegen humane reverse Transskriptase sowie HLA-DR, HLA B 3503 und CD 34 Antikörper wirken, oder die weiteren Antikörper monoklonale und/oder polyklonale Antikörper gegen die genannten Antigene sind.

40. Trägermaterial nach einem oder mehreren der Ansprüche 1 bis 39, dadurch gekennzeichnet, daß die weiteren Antikörper gegen humane reverse Transskriptase, CD 5 der T-Lymphozyten sowie gegen Epithel spezifisches Antigen (ESA) Antikörper wirken, oder die weiteren Antikörper monoklonale und/oder polyklonale Antikörper gegen die genannten Antigene sind.

41. Trägermaterial nach einem oder mehreren der Ansprüche 1 bis 39, dadurch gekennzeichnet, daß die weiteren Antikörper gegen humane reverse Transskriptase sowie gegen Promyelozyten vom Typ PG-M3 (Moab) und gegen den Retinoidsäure-Rezeptor alpha wirken, oder die weiteren Antikörper monoklonale und/oder polyklonale Antikörper gegen die genannten Antigene sind.

42. Trägermaterial nach einem oder mehreren der Ansprüche 1 bis 41, dadurch gekennzeichnet, daß die weiteren Antikörper gegen humane reverse Transskriptase sowie gegen Makrophagen vom Typ (MAb) sowie gegen Epithel spezifisches Antigen (ESA), Antihuman Episialin (EMA), Farnesyl Transferase, Cyclin dependant Kinase, E1-A-Protein ATM, MLH 1, XP-A, XP-B, MGMT, BLM, NF1 PTH, p27, RB und gegen Caspase Aktivatoren wirken, oder die weiteren Antikörper monoklonale und/oder polyklonale Antikörper gegen die genannten Antigene sind.

43. Trägermaterial nach einem oder mehreren der Ansprüche 1 bis 42, dadurch gekennzeichnet, daß die weiteren Antikörper gegen die reverse Transskriptase, das Protein A, Conductin-Protein, SDF-1-Protein, 6-C-Kine-Protein, MIP-3β-Protein und VCAM wirken, oder die weiteren Antikörper monoklonale und/oder polyklonale Antikörper gegen die genannten Antigene sind.

44. Trägermaterial nach einem oder mehreren der Ansprüche 1 bis 43, dadurch gekennzeichnet, daß die weiteren Antikörper gegen die reverse Transskriptase, das P-Glykoprotein (MDR) und P-Glykoprotein(MDR)-Fragmente wirken, oder die weiteren Antikörper monoklonale und/oder polyklonale Antikörper gegen die genannten Antigene sind.

45. Trägermaterial nach einem oder mehreren der Ansprüche 1 bis 44, dadurch gekennzeichnet, daß die weiteren Antikörper gegen die reverse Transskriptase, vaskulären endothelialen Wachstunsfaktor (VEGF) und (VEFG-Fragmente), (VEGF-Rezeptor Antagonisten) wirken, oder die weiteren Antikörper monoklonale und/oder polyklonale Antikörper gegen die genannten Antigene sind.

46. Trägermaterial nach einem oder mehreren der Ansprüche 1 bis 45, dadurch gekennzeichnet, daß die weiteren Antikörper gegen die reverse Transskriptase, gegen Epithel spezifisches Antigen (ESA), gegen die Matrixmetahloproteinase-2 (MMP-2) und/oder gegen den Aktivator der Matrixmetalloproteinase-1 (MT1-MMP) wirken, oder die weiteren Antikörper monoklonale und/oder polyklonale Antikörper gegen die genannten Antigene sind.

47. Trägermaterial nach einem oder mehreren der Ansprüche 1 bis 46, dadurch gekennzeichnet, daß die weiteren Antikörper gegen die bei der Gentherapie mit Oligodeoxynukleotiden im Rahmen Antisense Technik auftretenden Antikörper oder gegen die bei der Gentherapie der Genersatztherapie, der Gensequenzänderungstherapie oder gegen die bei der Virus Vektor Gentherapie oder gegen die bei der Virus Vektor Augmentationstherapie oder gegen die bei der Artifizieilen Chromosomen-Therapie oder gegen die bei der DNS-Plasmid-Therapie oder die bei der Immunogen-Therapie, gegen die bei der Tumor-Supressor-Onkogene-Apoptosis-Kombinationstherapie gegen die bei der Gentherapie mit Rekombinanten-Material entstehenden Antikörper wirken, oder die weiteren Antikörper monoklonale und/oder polyklonale Antikörper gegen die genannten Antigene sind.

48. Trägermaterial nach einem oder mehreren der Ansprüche 1 bis 47, dadurch gekennzeichnet, daß die weiteren Antikörper gegen die reverse Transskriptase, das Protein das P-Glykoprotein,gegen das Protein HER-2/neu ,gegen Antihuman Episialin (EMA) und gegen VCAM wirken, oder die weiteren Antikörper monoklonale und/oder polyklonale Antikörper gegen die genannten Antigene sind.

49. Trägermaterial nach einem oder mehreren der Ansprüche 1 bis 48, dadurch gekennzeichnet, daß die weiteren Antikörper gegen die reverse Transskriptase, das Protein HER-2/neu, MYC, ERB B1-3, RAS, MSH2, p53 und gegen VCAM wirken, oder die weiteren Antikörper monoklonale und/oder polyklonale Antikörper gegen die genannten Antigene sind.

50. Trägermaterial nach einem oder mehreren der Ansprüche 1 bis 49, dadurch gekennzeichnet, daß die weiteren Antikörper gegen die reverse Transskriptase, gegen die durch das Krebswachstum mutierten Proteine HER-2/neu, MYC, ERB B1-3, RAS, MSH2, p53 und/oder VCAM wirken, oder die weiteren Antikörper monoklonale und/oder polyklonale Antikörper gegen die genannten Antigene sind.

51. Trägermaterial nach einem oder mehreren der Ansprüche 1 bis 50, dadurch gekennzeichnet, daß die weiteren Antikörper gegen die reverse Transskriptase, gegen das durch das Krebswachstum mutierte Proteine, RAS, MSH2, p53 sowie VCAM wirken, oder die weiteren Antikörper monoklonale und/oder polyklonale Antikörper gegen die genannten Antigene sind.

52. Trägermaterial nach einem oder mehreren der Ansprüche 1 bis 51, dadurch gekennzeichnet, daß die weiteren Antikörper gegen das Endothelin (VEGF), (VEGF-Fragmente), (VEGF-Rezeptor Antagonisten), Matrix-Proteine, VCAM, gegen Catenin (alpha, beta), neurale Zelladhäsionsproteine und gegen Cadherine wirken, oder die weiteren Antikörper monoklonale und/oder polyklonale Antikörper gegen die genannten Antigene sind.

53. Trägermaterial nach einem oder mehreren der Ansprüche 1 bis 52, dadurch gekennzeichnet, daß die weiteren Antikörper gegen das Endothelin (VEGF), (VEGF-Fragmente), (VEGF-Rezeptor Antagonisten), gegen VCAM und HLA und gegen humane Leukozyten wirken, oder die weiteren Antikörper monoklonale und/oder polyklonale Antikörper gegen die genannten Antigene sind.

54. Trägermaterial nach einem oder mehreren der Ansprüche 1 bis 53, dadurch gekennzeichnet, daß die weiteren Antikörper gegen das Endothelin (VEGF), (VEGF-Fragmente), (VEGF-Rezeptor Antagonisten), (VEGF), (VEGF-Fragmente), (VEGF-Rezeptor Antagonisten), VCAM und gegen HLA und/oder gegen humane Leukozyten und/oder T-Lymphozyten wirken, oder die weiteren Antikörper monoklonale und/oder polyklonale Antikörper gegen die genannten Antigene sind.

55. Trägermaterial nach einem oder mehreren der Ansprüche 1 bis 54, dadurch gekennzeichnet, daß die weiteren Antikörper gegen das VCAM-Protein und/oder gegen Rezeptor für Glykat-Endprodukte und/oder gegen Hemozoin wirken, oder die weiteren Antikörper monoklonale und/oder polyklonale Antikörper gegen die genannten Antigene sind.

56. Trägermaterial nach einem oder mehreren der Ansprüche 1 bis 55, dadurch gekennzeichnet, daß die weiteren Antikörper gegen das lösliche Plasmodium falciparum Antigen, gegen das VCAM-Protein, gegen Rezeptor für Glykat-Endprodukte und gegen Hemozoin wirken, oder die weiteren Antikörper monoklonale und/oder polyklonale Antikörper gegen die genannten Antigene sind.

57. Trägermaterial nach einem oder mehreren der Ansprüche 1 bis 56, daß die weiteren Antikörper gegen das lösliche Plasmodium falciparum Antigen, gegen das VCAM- und ICAM-Protein, gegen den Rezeptor für Glykat-Endprodukte und gegen Hemozoin wirken, oder die weiteren Antikörper monoklonale und/oder polyklonale Antikörper gegen die genannten Antigene sind.

58. Trägermaterial nach einem oder mehreren der Ansprüche 1 bis 57, dadurch gekennzeichnet, daß die weiteren Antikörper gegen das Plasmodium falciparum Antigen, gegen die Glykosyl-phosphatidylinositol-Produkte (GPI) aus Plasmodium falciparum, aus Trypanosoma brucei oder aus Leishmania mexicana, gegen das VCAM- und ICAM-Protein, gegen den Rezeptor für Glykat-Endprodukte und gegen Hemozoin wirken, oder die weiteren Antikörper monoklonale und/oder polyklonale Antikörper gegen die genannten Antigene sind.

59. Trägermaterial nach einem oder mehreren der Ansprüche 1 bis 58, dadurch gekennzeichnet, daß die weiteren Antikörper gegen das lösliche Plasmodium falciparum, aus Trypanosoma brucei oder aus Leishmania mexicana, gegen das VCAM-und gegen das ICAM-Protein, gegen den Rezeptor für GlykatEndprodukte, gegen Hemozoin und gegen Lipopolysaccharide wirken, oder die weiteren Antikörper monoklonale und/oder polyklonale Antikörper gegen die genannten Antigene sind.

60. Trägermaterial nach einem oder mehreren der Ansprüche 1 bis 59, dadurch gekennzeichnet, daß die weiteren Antikörper gegen Hemozoin und gegen den Endothelrezeptor vom Typ PECAM-1/CD31 wirken, oder die weiteren Antikörper monoklonale und/oder polyklonale Antikörper gegen die genannten Antigene sind.

61. Trägermaterial nach einem oder mehreren der Ansprüche 1 bis 60, dadurch gekennzeichnet, daß die weiteren Antikörper gegen das Mycobakterium leprae, das Mitsuda Antigen und das Dharmendra Antigen wirken, oder die weiteren Antikörper monoklonale und/oder polyklonale Antikörper gegen die genannten Antigene sind.

62. Trägermaterial nach einem oder mehreren der Ansprüche 1 bis 61, dadurch gekennzeichnet, daß die weiteren Antikörper gegen das Mycobakterium leprae, das Mitsuda Antigen, das Dharmendra Antigen sowie gegen das VCAM- und ICAM-Protein wirken, oder die weiteren Antikörper monoklonale und/oder polyklonale Antikörper gegen die genannten Antigene sind.

63. Trägermaterial nach einem oder mehreren der Ansprüche 1 bis 62, dadurch gekennzeichnet, daß die weiteren Antikörper gegen das Protein A und gegen das Protein p59 fyn der Kinase wirken, oder die weiteren Antikörper monoklonale und/oder polyklonale Antikörper gegen die genannten Antigene sind.

64. Trägermaterial nach einem oder mehreren der Ansprüche 1 bis 63, dadurch gekennzeichnet, daß die weiteren Antikörper gegen die reverse humane Transskriptase, gegen das Protein A, gegen das Protein p59 fyn der Kinase und gegen das Aktivator-Protein-1 (AP-1) wirken, oder die weiteren Antikörper monoklonale und/oder polyklonale Antikörper gegen die genannten Antigene sind.

65. Trägermaterial nach einem oder mehreren der Ansprüche 1 bis 64, dadurch gekennzeichnet, daß die weiteren Antikörper gegen die reverse humane Transskriptase, gegen das Protein A, gegen das Protein p59 fyn der Kinase, gegen das Aktivator-Protein-1 (AP-1) und gegen das Serum Amyloid-A-Protein (SAA) wirken, oder die weiteren Antikörper monoklonale und/oder polyklonale Antikörper gegen die genannten Antigene sind.

66. Trägermaterial nach einem oder mehreren der Ansprüche 1 bis 65, dadurch gekennzeichnet, daß die weiteren Antikörper gegen die reverse humane Transskriptase, gegen das Protein A, gegen das Protein p59 fyn der Kinase, gegen das Aktivator-Protein-1 (AP-1), gegen das Serum Amyloid-A-Protein (SAA), Plasminogen-Aktivator-Inhibitor-Typ-1-Antigen (PAI-1) sowie gegen Farnesyl Transferase, Cyclin dependant Kinase und Caspase Aktivatoren wirken, oder die weiteren Antikörper monoklonale und/oder polyklonale Antikörper gegen die genannten Antigene sind.

67. Trägermaterial nach einem oder mehreren der Ansprüche 1 bis 65, dadurch gekennzeichnet, daß die weiteren Antikörper gegen die reverse humane Transskriptase, Protein-1 (AP-1), gegen das Serum beta-Amyloid-Protein, beta-Amyloid-Protein-Fragmente, beta-Amyloid-Präkursor-Protein (beta APP) und gegen das Oncostatin-M-Protein (OSM) wirken, oder die weiteren Antikörper monoklonale und/oder polyklonale Antikörper gegen die genannten Antigene sind.

68. Trägermaterial nach einem oder mehreren der Ansprüche 1 bis 67, dadurch gekennzeichnet, daß die weiteren Antikörper gegen die reverse humane Transskriptase, gegen das mutierte p53-Protein und gegen das Ki.67-Antigen, sowie gegen Farnesyl Transferase, Cyclin dependant Kinase und Gaspase Aktivatoren wirken, oder die weiteren Antikörper monoklonale und/oder polyklonale Antikörper gegen die genannten Antigene sind.

69. Trägermaterial nach einem oder mehreren der Ansprüche 1 bis 68, dadurch gekennzeichnet, daß die weiteren Antikörper gegen die reverse humane Transskriptase, gegen das mutierte p53-Protein und gegen die, die bei Anwendung von Gen-transfizierten Cholesterol-Liposomen gegen Tumore entstehen, wirken, oder die weiteren Antikörper monoklonale und/oder polyklonale Antikörper gegen die genannten Antigene sind.

70. Trägermaterial nach einem oder mehreren der Ansprüche 1 bis 69, dadurch gekennzeichnet, daß die weiteren Antikörper gegen die reverse Transskriptase, gegen das mutierte p53-Protein und gegen den vakulären endothelialen Gefäßwachstumstaktor (VEGF), (VEGF-Fragmente), (VEGF-Rezeptor Antagonisten), sowie gegen Conductin und P-Selectin wirken, oder die weiteren Antikörper monoklonale und/oder polyklonale Antikörper gegen die genannten Antigene sind.

71. Trägermaterial nach einem oder mehreren der Ansprüche 1 bis 70, dadurch gekennzeichnet, daß die weiteren Antikörper gegen die reverse humane Transskriptase, gegen das beta-2-Mikroglobulin (B2-MG) und gegen den vakulären endotheliaien Gefäßwachstumsfaktor (VEGF), (VEGF-Fragmente), (VEGF-Rezeptor Antagonisten), sowie gegen Farnesyl Transferase, Cyclin dependant Kinase und Gaspase Aktivatoren wirken, oder die weiteren Antikörper monoklonale und/oder polyklonale Antikörper gegen die genannten Antigene sind.

72. Trägermaterial nach einem oder mehreren der Ansprüche 1 bis 71, dadurch gekennzeichnet, daß die weiteren Antikörper gegen das beta-2-Mikroglobulin (B2-MG), gegen den vakulären endothelialen Wachstumsfaktor Endothelin (VEGF), (VEGF-Fragmente), (VEGF-Rezeptor Antagonisten), sowie gegen Farnesyl Transferase, Cyclin dependant Kinase und Caspase Aktivatoren vom monoklonalen Typ wirken, oder die weiteren Antikörper monoklonale und/oder polyklonale Antikörper gegen die genannten Antigene sind.

73. Trägermaterial nach einem oder mehreren der Ansprüche 1 bis 72, dadurch gekennzeichnet, daß die weiteren Antikörper gegen den Plasma Endothelin (VEGF), (VEGF-Fragmente), (VEGF-Rezeptor Antagonisten) (VEGF), gegen das Oncostatin Protein und gegen Interleukin 6 wirken, oder die weiteren Antikörper monoklonale und/oder polyklonale Antikörper gegen die genannten Antigene sind.

74. Trägermaterial nach einem oder mehreren der Ansprüche 1 bis 73, dadurch gekennzeichnet, daß die weiteren Antikörper gegen die reverse humane Transskriptase und gegen das Rezeptor Antigen CD 4 wirken, oder die weiteren Antikörper monoklonale und/oder polyklonale Antikörper gegen die genannten Antigene sind.

75. Trägermaterial nach einem oder mehreren der Ansprüche 1 bis 74, dadurch gekennzeichnet, daß die weiteren Antikörper gegen reverse humane Transskriptase, gegen das Rezeptor Antigen CD 4 vom monoklonalen Typ und gegen HIV-Protease-Inhibitoren wirken, oder die weiteren Antikörper monoklonale und/oder polyklonale Antikörper gegen die genannten Antigene sind.

76. Trägermaterial nach einem oder mehreren der Ansprüche 1 bis 75, dadurch gekennzeichnet, daß die weiteren Antikörper gegen reverse humane Transskriptase, gegen das Rezeptor Antigen CD 4 und gegen das p24 Antigen wirken, oder die weiteren Antikörper monoklonale und/oder polyklonale Antikörper gegen die genannten Antigene sind.

77. Trägermaterial nach einem oder mehreren der Ansprüche 1 bis 76, dadurch gekennzeichnet, daß die weiteren Antikörper gegen das Mycobakterium tuberkulosis und das BCG-Antigen wirken, oder die weiteren Antikörper monoklonale und/oder polyklonale Antikörper gegen die genannten Antigene sind.

78. Trägermaterial nach einem oder mehreren der Ansprüche 1 bis 77, dadurch gekennzeichnet, daß die weiteren Antikörper gegen Mycobakterium tuberkulosis, das BCG-Antigen, Endothelin (VEGF), (VEGF-Fragmente), (VEGF-Rezeptor Antagonisten), gegen VCAM, gegen Telomerase sowie gegen Farnesyl Transferase, Cyclin dependant Kinase und Caspase Aktivatoren wirken, oder die weiteren Antikörper monoklonale und/oder polyklonale Antikörper gegen die genannten Antigene sind.

79. Trägermaterial nach einem oder mehreren der Ansprüche 1 bis 78, dadurch gekennzeichnet, daß die weiteren Antikörper gegen das Endothelin (VEGF), (VEGF-Fragmente), (VEGF-Rezeptor Antagonisten)-Protein, VCAM, gegen HLA, gegen humane Leukozyten, gegen T-Lymphozyten und gegen das P-Selectin wirken, oder die weiteren Antikörper monoklonale und/oder polyklonale Antikörper gegen die genannten Antigene sind.

80. Trägermaterial nach einem oder mehreren der Ansprüche 1 bis 79, dadurch gekennzeichnet, daß die weiteren Antikörper gegen HLA, gegen humane Leukozyten, T-Lymphozyten, gegen extrazelluläre Matrix und gegen das P-Selectin wirken, oder die weiteren Antikörper monoklonale und/oder polyklonale Antikörper gegen die genannten Antigene sind.

81. Trägermaterial nach einem oder mehreren der Ansprüche 1 bis 80, dadurch gekennzeichnet, daß die weiteren Antikörper gegen das eosinophile kationische Protein (ECP) vom monoklonalen Typ wirken.

82. Trägermaterial nach einem oder mehreren der Ansprüche 1 bis 81, dadurch gekennzeichnet, daß die weiteren Antikörper gegen die reverse humane Transskriptase, gegen das mutierte p53-Protein und gegen das Eppstein-Barr-Virus und gegen das E1A-Virusprotein wirken, oder die weiteren Antikörper monoklonale und/oder polyklonale Antikörper gegen die genannten Antigene sind.

83. Trägermaterial nach einem oder mehreren der Ansprüche 1 bis 82, dadurch gekennzeichnet, daß die weiteren Antikörper gegen das Endothelin (VEGF), (VEGF-Fragmente), (VEGF-Rezeptor Antagonisten), VCAM und gegen das HLA-B-7-Residuum 75-84 der alpha-Domäne, gegen humane Leukozyten, T-Lymphozyten und gegen das P-Selectin wirken, oder die weiteren Antikörper monoklonale und/oder polyklonale Antikörper gegen die genannten Antigene sind.

84. Trägermaterial nach einem oder mehreren der Ansprüche 1 bis 83, dadurch gekennzeichnet, daß die weiteren Antikörper gegen das Endothelin (VEGF), (VEGF-Fragmente), (VEGF-Rezeptor Antagonisten), VCAM und gegen das HLA-B-7-Residuum 75-84 der alpha-Domäne wirken, oder die weiteren Antikörper monoklonale und/oder polyklonale Antikörper gegen die genannten Antigene sind.

85. Trägermaterial nach einem oder mehreren der Ansprüche 1 bis 84, dadurch gekennzeichnet, daß die weiteren Antikörper gegen das Endothelin (VEGF), (VEGF-Fragmente), (VEGF-Rezeptor Antagonisten), VCAM und gegen A-beta-2-M-Amyloid wirken, oder die weiteren Antikörper monoklonale und/oder polyklonale Antikörper gegen die genannten Antigene sind.

86. Trägermaterial nach einem oder mehreren Ansprüche 1 bis 85, dadurch gekennzeichnet, daß die weiteren Antikörper gegen das Endothelin (VEGF), (VEGF-Fragmente), (VEGF-Rezeptor Antagonisten), VCAM und gegen A-beta-2-M-Amyloid, Conductin-Protein, SDF-1-Protein, 6-C-Kine-Protein und MIP-3β-Protein wirken, oder die weiteren Antikörper monoklonale und/oder polyklonale Antikörper gegen die genannten Antigene sind.

87. Trägermaterial nach einem oder mehreren der Ansprüche 1 bis 86, dadurch gekennzeichnet, daß die weiteren Antikörper gegen das A-beta-2-M-Amyloid-Selectin (P,L), CD 994, NKG 2A BCL2, Endothelin (VEGF) und (VEGF-Fragmente), (VEGF-Rezeptor Antagonisten) wirken, oder die weiteren Antikörper monoklonale und/oder polyklonale Antikörper gegen die genannten Antigene sind.

88. Trägermaterial nach einem oder mehreren der Ansprüche 1 bis 87, dadurch gekennzeichnet, daß die weiteren Antikörper gegen Gallensäuren, Ammoniak und Bilirubin wirken, oder die weiteren Antikörper monoklonale und/oder polyklonale Antikörper gegen die genannten Antigene sind.

89. Trägermaterial nach einem oder mehreren der Ansprüche 1 bis 88, dadurch gekennzeichnet, daß die weiteren Antikörper gegen CD 3, LAF, A-beta-2-M-Amyloid-Selectin (P,L), CD 994, NKG 2A BCL2, Endothelin (VEGF), (VEGF-Fragmente) und (VEGF-Rezeptor Antagonisten) wirken, oder die weiteren Antikörper monoklonale und/oder polyklonale Antikörper gegen die genannten Antigene sind.

90. Trägermaterial nach einem oder mehreren der Ansprüche 1 bis 89, dadurch gekennzeichnet, daß die weiteren Antikörper gegen CD 994, NKG 2A, Endothelin (VEGF), (VEGF-Fragmente) und (VEGF-Rezeptor Antagonisten) wirken, oder die weiteren Antikörper monoklonale und/oder polyklonale Antikörper gegen die genannten Antigene sind.

91. Trägermaterial nach einen oder mehreren der Ansprüche 1 bis 90, dadurch gekennzeichnet, daß die weiteren Antikörper gegen CD 3 und LFA-1-alpha wirken, oder die weiteren Antikörper monoklonale und/oder polyklonale Antikörper gegen die genannten Antigene sind.

92. Trägermaterial nach einem oder mehreren der Ansprüche 1 bis 91, dadurch gekennzeichnet, daß die weiteren Antikörper gegen MDR 1-Protein vom Typ MRK 16 und C 219, Endothelin (VEGF), (VEGF-Fragmente), (VEGF-Rezeptor Antagonisten), Protein A und P-Selectin wirken, oder die weiteren Antikörper monoklonale und/oder polyklonale Antikörper gegen die genannten Antigene sind.

93. Trägermaterial nach einem oder mehreren der Ansprüche 1 bis 93, dadurch gekennzeichnet, daß die weiteren Antikörper gegen MDR1-Protein vom Typ MRK 16 und C 219, Endoethlin (VEGF), (VEGF-Fragmente), (VEGF-Rezeptor Antagonisten), (VEGF), (VEGF-Fragmente) und (VEGF-Rezeptor Antagonisten) -Protein wirken, oder die weiteren Antikörper monoklonale und/oder polyklonale Antikörper gegen die genannten Antigene sind.

94. Trägermaterial nach einem oder mehreren der Ansprüche 1 bis 93, dadurch gekennzeichnet, daß die weiteren Antikörper gegen die Antikörper des Fusionsproteins aus Fab Teil (aus dem monoklonalen Antikörper C 215) und dem -SEA (Staphyiokokken Enterotoxin Antigen), Endothelin (VEGF), (VEGF-Fragmente) und (VEGF-Rezeptor Antagonisten) wirken, oder die weiteren Antikörper monoklonale und/oder polyklonale Antikörper gegen die genannten Antigene sind.

95. Trägermaterial nach einem oder mehreren der Ansprüche 1 bis 94, dadurch gekennzeichnet, daß die weiteren Antikörper gegen Epidermal Growth Faktor (EGFR), Endothelin (VEGF), (VEFG-Fragmente), (VEGF-Rezeptor Antagonisten), gegen Farnesyl Transferase, Cyclin dependant Kinase (CDK), gegen Caspase Aktivatoren und Selectin (P,L) wirken, oder die weiteren Antikörper monoklonale und/oder polyklonale Antikörper gegen die genannten Antigene sind.

96. Trägermaterial nach einem oder mehreren der Ansprüche 1 bis 95, dadurch gekennzeichnet, daß die weiteren Antikörper gegen Epidermal Growth Faktor (EGFR), Endothelin (VEGF), (VEGF-Fragmente), (VEGF-Rezeptor Antagonisten) (VEGF), (VEGF-Fragmente), (VEGF-Rezeptor Antagonisten) - Protein, Selectin (P,L), gegen Interleukin 6 oder gegen Interleukin Gen exprimierende Konstrukte wirken, oder die weiteren Antikörper monoklonale und/oder polyklonale Antikörper gegen die genannten Antigene sind.

97. Trägermaterial nach einem oder mehreren der Ansprüche 1 bis 96, dadurch gekennzeichnet, daß die weiteren Antikörper gegen Epidermal Growth Faktor (EGFR), Endothelin (VEGF), (VEGF-Fragmente), (VEGF-Rezeptor Antagonisten) (VEGF), (VEGF-Fragmente), (VEGF-Rezeptor Antagonisten)-Protein, Selectin (P,L) und OX 40 wirken, oder die weiteren Antikörper monoklonale und/oder polyklonale Antikörper gegen die genannten Antigene sind.

98. Trägermaterial nach einem oder mehreren der Ansprüche 1 bis 97, dadurch gekennzeichnet, daß die weiteren Antikörper gegen HLA B 3503 wirken.

99. Trägermaterial nach einem oder mehreren der Ansprüche 1 bis 98, dadurch gekennzeichnet, daß die weiteren Antikörper gegen Interleukin-8 wirken.

100. Trägermaterial nach einem oder mehreren der Ansprüche 1 bis 99, dadurch gekennzeichnet, daß die weiteren Antikörper gegen Epidermal Growth Faktor (EGFR), (Endothelin (VEGF), (VEGF-Fragmente), (VEGF-Rezeptor Antagonisten), Selectin (P,L) und Interleukin-1 wirken, oder die weiteren Antikörper monoklonale und/oder polyklonale Antikörper gegen die genannten Antigene sind.

101. Trägermaterial nach einen oder mehreren der Ansprüche 1 bis 100, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und Interleukin-2-Rezeptor wirken, oder die weiteren Antikörper monoklonale und/oder polyklonale Antikörper gegen die genannten Antigene sind.

102. Trägermaterial nach einem oder mehreren der Ansprüche 1 bis 101, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und Squamöses Zell-Karzinom-Antigen (SCC) wirken, oder die weiteren Antikörper monoklonale und/oder polyklonale Antikörper gegen die genannten Antigene sind.

103. Trägermaterial nach einem oder mehreren der Ansprüche 1 bis 102, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und Matrix-Protein-Tenascin-C (TN-C) wirken, oder die weiteren Antikörper monoklonale und/oder polyklonale Antikörper gegen die genannten Antigene sind.

104. Trägermaterial nach einem oder mehreren der Ansprüche 1 bis 103, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und gegen Sialyl Lewis x wirken, oder die weiteren Antikörper monoklonale und/oder polyklonale Antikörper gegen die genannten Antigene sind.

105. Trägermaterial nach einem oder mehreren der Ansprüche 1 bis 104, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und gegen IGM-Zytostatica Kombinationen wirken, oder die weiteren Antikörper monoklonale und/oder polyklonale Antikörper gegen die genannten Antigene sind.

106. Trägermaterial nach einem oder mehreren der Ansprüche 1 bis 105, dadurch gekennzeichnet, daß die weiteren Antikörper gegen Protein A, gegen Human HLA Class I Antigen, gegen Anti-Thrombopeotin, Lipopolysaccharid und gegen Van Glykoprotein wirken, oder die weiteren Antikörper monoklonale und/oder polyklonale Antikörper gegen die genannten Antigene sind.

107. Trägermaterial nach einem oder mehreren der Ansprüche 1 bis 106, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und NF-kappa-B-Gen-exprimierende Konstrukte wirken, oder die weiteren Antikörper monoklonale und/oder polyklonale Antikörper gegen die genannten Antigene sind.

108. Trägermaterial nach einem oder mehreren der Ansprüche 1 bis 107, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und Endothel-aktivierendes Polypeptid II (EMAP II) wirken, oder die weiteren Antikörper monoklonale und/oder polyklonale Antikörper gegen die genannten Antigene sind.

109. Trägermaterial nach einem oder mehreren der Ansprüche 1 bis 108, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und das Antiprotein gegen den Endothel aktivierendes Polypeptid II (EMAP II) wirken, oder die weiteren Antikörper monoklonale und/oder polyklonale Antikörper gegen die genannten Antigene sind.

110. Trägermaterial nach einem oder mehreren der Ansprüche 1 bis 109, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L), Anti CD3 und Glykoprotein EGP2 als bispezifischem Antikörper wirken, oder die weiteren Antikörper monoklonale und/oder polyklonale Antikörper gegen die genannten Antigene sind.

111. Trägermaterial nach einem oder mehreren der Ansprüche 1 bis 110, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L), Makrophagen-Migration-inhibitorischem Faktor (-MIF) und dessen Antikörper (Anti -MIF), Protein A, ConductinProtein, SDF-1-Protein, 6-C-Kine-Protein und MIP-3β wirken, oder die weiteren Antikörper monoklonale und/oder polyklonale Antikörper gegen die genannten Antigene sind.

112. Trägermaterial nach einem oder mehreren der Ansprüche 1 bis 111, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und Leukämie-inhibitorischen Faktor(LIF) und dessen Antikörper (Anti LIF) wirken, oder die weiteren Antikörper monoklonale und/oder polyklonale Antikörper gegen die genannten Antigene sind.

113. Trägermaterial nach einem oder mehreren der Ansprüche 1 bis 112, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L)wirken und Anti-N-ramp-Gen-Konstrukt wirken, oder die weiteren Antikörper monoklonale und/oder polyklonale Antikörper gegen die genannten Antigene sind.

114. Trägermaterial nach einem oder mehreren der Ansprüche 1 bis 113, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L), Substanz P (SP) und Substanz-SP-Fragmente wirken, oder die weiteren Antikörper monoklonale und/oder polyklonale Antikörper gegen die genannten Antigene sind.

115. Trägermaterial nach einem oder mehreren der Ansprüche 1 bis 114, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L) und Interleukin-1-Rezeptor wirken, oder die weiteren Antikörper monoklonale und/oder polyklonale Antikörper gegen die genannten Antigene sind.

116. Trägermaterial nach einem oder mehreren der Ansprüche 1 bis 115, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L), Interleukin 2 und Interleukin 4 wirken, oder die weiteren Antikörper monoklonale und/oder polyklonale Antikörper gegen die genannten Antigene sind.

117. Trägermaterial nach einem oder mehreren der Ansprüche 1 bis 116, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L), Substanz P (SP) und vasoaktive intestinale Peptide (VIP), Protein A, Conductin-Protein, SDF-1-Protein, 6-C-Kine-Protein, MIP-3β wirken, oder die weiteren Antikörper monoklonale und/oder polyklonale Antikörper gegen die genannten Antigene sind.

118. Trägermaterial nach einem oder mehreren der Ansprüche 1 bis 117, dadurch gekennzeichnet, daß die weiteren Antikörper gegen Epidermal Growth Faktor (EGFR), Selectin (P,L), (VEFG), (VEFG-Fragmente), (VEFG-Rezeptor Antagonisten), Farnesyl Transferase, Cyclin dependant Kinase und Proleration wirken, oder die weiteren Antikörper monoklonale und/oder polyklonale Antikörper gegen die genannten Antigene sind.

119. Trägermaterial nach einem oder mehreren der Ansprüche 1 bis 118, dadurch gekennzeichnet, daß die weiteren Antikörper gegen die reverse Transskriptase, gegen die durch das Krebswachstum mutierten Proteine HER-2-neu, MYC, ERB B1-3, RAS, p53, gegen VCAM, gegen Epidermal Growth Faktor (EGFR), Selectin (P,L), (VEGF), (VEGF-Fragmente und (VEGF-Rezeptor Antagonisten) wirken, oder die weiteren Antikörper monoklonale und/oder polyklonale Antikörper gegen die genannten Antigene sind.

120. Trägermaterial nach einem oder mehreren Ansprüche 1 bis 119, dadurch gekennzeichnet, daß die weiteren Antikörper gegen P-Glykoprotein und P-Glykoprotein-Fragmente, gegen Endorphine, Enkephaline, Melanozyten stimulierende Hormone, Opiod, Substanz P und gegen deren Peptide und Rezeptorantagonisten und Antagonisten wirken, oder die weiteren Antikörper monoklonale und/oder polyklonale Antikörper gegen die genannten Antigene sind.

121. Trägermaterial nach einem oder mehreren der Ansprüche 1 bis 120, dadurch gekennzeichnet, daß die weiteren Antikörper gegen CD 4, gegen den MHC II (Major Histokompatibilitätskomplex)-Peptid-Komplex und gegen LFA-1 (Leukozyten-Funktion assoziiertes Antigen) wirken, oder die weiteren Antikörper monoklonale und/oder polyklonale Antikörper gegen die genannten Antigene sind.

122. Trägermaterial nach einem oder mehreren der Ansprüche 1 bis 121, dadurch gekennzeichnet, daß die weiteren Antikörper gegen das lösliche Plasmodium falciparum Antigen, gegen das VCAM-Protein und/oder gegen Rezeptor für Glykat-Endprodukte und/oder gegen das cp 26 und cp 29, Circumsporozoit Proteins des Plasmodium falciparum und/oder gegen Hemozoin wirken, oder die weiteren Antikörper monoklonale und/oder polyklonale Antikörper gegen die genannten Antigene sind.

123. Trägermaterial nach einem oder mehreren der Ansprüche 1 bis 122, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L), Endothel aktivierendes Polypeptid II (EMAP II), Protein A, Conductin-Protein, SDF-1-Protein, 6-C-Kine-Protein und MIP-3β wirken, oder die weiteren Antikörper monoklonale und/oder polyklonale Antikörper gegen die genannten Antigene sind.

124. Trägermaterial nach einem oder mehreren der Ansprüche 1 bis 123, dadurch gekennzeichnet, daß die weiteren Antikörper gegen CD 4 und gegen MHC II (Major Hisotkompatibilitätskomplex)-Peptid-Komplex wirken, oder die weiteren Antikörper monoklonale und/oder polyklonale Antikörper gegen die genannten Antigene sind.

125. Trägermaterial nach einem oder mehreren der Ansprüche 1 bis 124, dadurch gekennzeichnet, daß die weiteren Antikörper gegen CD 8 und gegen MHC I (Major Histokompatibilitätskompiex)-Peptid-Komplex wirken, oder die weiteren Antikörper monoklonale und/oder polyklonale Antikörper gegen die genannten Antigene sind.

126. Trägermaterial nach einem oder mehreren der Ansprüche 1 bis 125, dadurch gekennzeichnet, daß die weiteren Antikörper gegen Epidermal Growth Faktor (EGFR), Selectin (P,L), (VEGF), (VEGF-Fragmente), (VEGF-Rezeptor Antagonisten) und Neuropilin als spezifischen Inhibitor der Gefäßneublidung und endothelialen Proliferation wirken, oder die weiteren Antikörper monoklonale und/oder polyklonale Antikörper gegen die genannten Antigene sind.

127. Trägermaterial nach einem oder mehreren der Ansprüche 1 bis 126, dadurch gekennzeichnet, daß die weiteren Antikörper gegen HLA-DR 1,2,4 und gegen Oligodendrozyten Glykoprotein und gegen Semaphorin als Antikörper vom monoklonalen Typ (MOG) wirken, oder die weiteren Antikörper monoklonale und/oder polyklonale Antikörper gegen die genannten Antigene sind.

128. Trägermaterial nach einem oder mehreren der Ansprüche 1 bis 127, dadurch gekennzeichnet, daß die weiteren Antikörper gegen HLA-DR 1,2,4, gegen Oligodendrozyten Glykoprotein (MOG), gegen das Epstein-Barr-Virus (EBV) und ggen Semaphorin Protein wirken, oder die weiteren Antikörper monoklonale und/oder polyklonale Antikörper gegen die genannten Antigene sind.

129. Trägermaterial nach einem oder mehreren der Ansprüche 1 bis 128, dadurch gekennzeichnet, daß die weiteren Antikörper gegen die reverse Transskriptase, gegen die durch das Krebswachstum mutierten Proteine HER-2-neu, MYC, ERB B1-3, RAS, MSH2, p 53, VCAM, Epidermal Growth Faktor (EGFR), Selectin (P,L), Sphingosine-1-Phosphate (SPP), (VEGF), (VEFG-Fragmente) und (VEFG-Rezeptor Antagonisten) vom monoklonalen Typ wirken, oder die weiteren Antikörper monoklonale und/oder polyklonale Antikörper gegen die genannten Antigene sind.

130. Trägermaterial nach einem oder mehreren der Ansprüche 1 bis 129, dadurch gekennzeichnet, daß die weiteren Antikörper gegen die reverse Transskriptase, gegen die das Krebswachstum blockierenden Faktoren, gegen HER-2-neu, MYC, ERB B1-3, RAS, MSH2, p53, VCAM, Epidermal Growth Faktor (EGFR), Selectin (P,L), (VEFG), (VEFG-Fragmente), (VEFG-Rezeptor Antagonisten), Farnesyl Transferase und Cyclin dependant Kinase (CDK) vom monoklonalen Typ wirken, oder die weiteren Antikörper monoklonale und/oder polyklonale Antikörper gegen die genannten Antigene sind.

131. Trägermaterial nach einem oder mehreren der Ansprüche 1 bis 130, dadurch gekennzeichnet, daß die weiteren Antikörper gegen die reverse Transskriptase, gegen die durch das Krebswachstum mutierten Proteine HER-2-neu, BRCA 1, BRCA 2, RAS, MSH2, p 53, Farnesyl Transferase, Cyclin dependant Kinase und/oder VCAM, Epidermal Growth Faktor (EGFR), Selectin (P,L), (VEGF), (VEFG-Fragmente) und (VEFG-Rezeptor Antagonisten vom monoklonalen Typ wirken, oder die weiteren Antikörper monoklonale und/oder polyklonale Antikörper gegen die genannten Antigene sind.

132. Trägermaterial nach einem oder mehreren der Ansprüche 1 bis 131, dadurch gekennzeichnet, daß die weiteren Antikörper gegen Epidermal Growth Faktor (EGFR), Endothelin (VEGF), (VEGF-Fragmente), (VEGF-Rezeptor Antagonisten), Selectin (P,L), Endothel aktivierendes Polypeptid II (EMAP II), Conductin-Protein, SDF-1-Protein und 6-C-Kine-Protein wirken, oder die weiteren Antikörper monoklonale und/oder polyklonale Antikörper gegen die genannten Antigene sind.

133. Trägermaterial nach einem oder mehreren der Ansprüche 1 bis 132, dadurch gekennzeichnet, daß die weiteren Antikörper gegen die reverse Transskriptase, gegen die durch das Krebswachstum mutierten Proteine HER-2-neu, BRCA 1, BRCA 2, RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor-Antagonisten) und E1-A-Protein vom monoklonalen Typ wirken, oder die weiteren Antikörper monoklonale und/oder polyklonale Antikörper gegen die genannten Antigene sind.

134. Trägermaterial nach einem oder mehreren der Ansprüche 1 bis 133, dadurch gekennzeichnet, daß die weiteren Antikörper gegen CD 4, gegen CD 8, gegen den MHC II (Major Histokompatibilitätskomplex)-Peptid-Komplex, MHC-I-Peptidkomplex und gegen Peptidyl Caspase-Inhibitoren wirken, oder die weiteren Antikörper monoklonale und/oder polyklonale Antikörper gegen die genannten Antigene sind.

135. Trägermaterial nach einem oder mehreren der Ansprüche 1 bis 134, dadurch gekennzeichnet, daß die weiteren Antikörper gegen die reverse Transskriptase, gegen die durch das Krebswachstum mutierten Proteine HER-2-neu, BRCA 1, BRCA 2, RAS, MSH 2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein und gegen Caspase Aktivatoren vom monoklonalen Typ wirken, oder die weiteren Antikörper monoklonale und/oder polyklonale Antikörper gegen die genannten Antigene sind.

136. Trägermaterial nach einem oder mehreren der Ansprüche 1 bis 135, dadurch gekennzeichnet, daß die weiteren Antikörper gegen HLA-DR 1,2,4- gegen Oligodendrozyten Glykoprotein (MOG), gegen das Epstein-Barr-Virus (EBV), Polypeptid Neurotoxin und gegen Sempaphorin Protein als Antikörper vom monoklonalen Typ, oder die weiteren Antikörper monoklonale und/oder polyklonale Antikörper gegen die genannten Antigene sind.

137. Trägermaterial nach einem oder mehreren der Ansprüche 1 bis 136, dadurch gekennzeichnet, daß die weiteren Antikörper gegen HLA-DR 1,2,4- gegen Oligodendrozyten Glykoprotein (MOG), gegen das Epstein-Barr-Virus (EBV), gegen Mikrotubulin assoziierte Proteine (MAPe), (MAPs, Tau-Proteine) und gegen Sempaphorin Protein als Antikörper vom monoklonalen Typ wirken, oder die weiteren Antikörper monoklonale und/oder polyklonale Antikörper gegen die genannten Antigene sind.

138. Trägermaterial nach einem oder mehreren der Ansprüche 1 bis 137, dadurch gekennzeichnet, daß die weiteren Antikörper gegen CD 4, gegen den MHC II (Major Histokompatibilitätskomplex)-Peptid-Komplex, gegen LFA-1 (Leukozyten-Funktion assoziiertes Antigen) und gegen HIV-Proteinase-Inhibitor wirken, oder die weiteren Antikörper monoklonale und/oder polyklonale Antikörper gegen die genannten Antigene sind.

139. Trägermaterial nach einem oder mehreren der Ansprüche 1 bis 138, dadurch gekennzeichnet, daß die weiteren Antikörper gegen CD 4, gegen den MHC II (Major Histokompatibilitätskomplex)-Peptid-Komplex, gegen LFA-1 (Leukozyten-Funktion assoziiertes Antigen), gegen ZIV-Proteinase, gegen Integrin und gegen Tristetraprolin wirken, oder die weiteren Antikörper monoklonale und/oder polyklonale Antikörper gegen die genannten Antigene sind.

140. Trägermaterial nach einem oder mehreren der Ansprüche 1 bis 139, dadurch gekennzeichnet, daß die weiteren Antikörper gegen CD 4, gegen den MHC II (Major Histokompatibilitätskomplex)-Peptid-Komplex, gegen LFA-1 (Leukozyten-Funktion assoziiertes Antigen), gegen HIV-Proteinase-Inhibitor, gegen Integrin und Viruscapside, Glykopeptide wirken, oder die weiteren Antikörper monoklonale und/oder polyklonale Antikörper gegen die genannten Antigene sind.

141. Trägermaterial nach einem oder mehreren der Ansprüche 1 bis 140, dadurch gekennzeichnet, daß die weiteren Antikörper gegen die reverse Transskriptase, gegen die durch das Krebswachstum mutierten Proteine HER-2-neu, BRCA 1, BRCA 2, RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein ATM, MLH 1, XP-A, XP-B, MGMT, BLM, NF1 PTH, p27, RB und gegen Caspase Aktivatoren vom monoklonalen Typ wirken, oder die weiteren Antikörper monoklonale und/oder polyklonale Antikörper gegen die genannten Antigene sind.

142. Trägermaterial nach einem oder mehreren der Ansprüche 1 bis 141, dadurch gekennzeichnet, daß die weiteren Antikörper gegen Kreatinin, Harnsäure, Harnstoff und Lipide und gegen beta-Mikroglobulin und Amyloid wirken, oder die weiteren Antikörper monoklonale und/oder polyklonale Antikörper gegen die genannten Antigene sind.

143. Trägermaterial nach einem oder mehreren der Ansprüche 1 bis 142, dadurch gekennzeichnet, daß die weiteren Antikörper gegen Cholera Toxin, Pseudomonas Exotoxin A, Staphylococcus alpha Toxin und Staphylococcus Enterotoxin A und B wirken, oder die weiteren Antikörper monoklonale und/oder polyklonale Antikörper gegen die genannten Antigene sind.

144. Trägermaterial nach einem oder mehreren der Ansprüche 1 bis 143, dadurch gekennzeichnet, daß die weiteren Antikörper gegen die reverse Transskriptase, gegen die durch das Krebswachstum mutierten Proteine HER-2-neu, Epithel spezifisches Antigen (ESA), RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein ATM, MLH 1, XP-A, XP-B, MGMT, BLM, NF1 PTH, p27, RB und gegen Caspase Aktivatoren vom monoklonalen Typ wirken, oder die weiteren Antikörper monoklonale und/oder polyklonale Antikörper gegen die genannten Antigene sind.

145. Trägermaterial nach einem oder mehreren der Ansprüche 1 bis 144, dadurch gekennzeichnet, daß die weiteren Antikörper gegen die reverse Transskriptase, gegen die durch das Krebswachstum mutierten Proteine HER-2-neu, Epithel spezifisches Antigen (ESA), Antihuman Episialin (EMA), RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein ATM, MLH 1, XP-A, XP-B, MGMT, BLM, NF1 PTH, p27, RB und gegen Caspase Aktivatoren vom monoklonalen Typ wirken, oder die weiteren Antikörper monoklonale und/oder polyklonale Antikörper gegen die genannten Antigene sind.

146. Trägermaterial nach einem oder mehreren der Ansprüche 1 bis 145, dadurch gekennzeichnet, daß die weiteren Antikörper gegen die reverse Transskriptase, gegen Proteine HER-2-neu, Epithel spezifisches Antigen (ESA), Antihuman Episialin (EMA), RAS, MSH2, p 53, Farnesyl Transferase, Cyclin dependant Kinase und/oder VCAM Epidermal Growth Faktor (EGFR), Selectin (P,L), (VEGF), (VEFG-Fragmente), (VEFG-Rezeptor Antagonisten), E1-A-Protein ATM, MLH 1, XP-A, XP-B, MGMT, BLM, NF1 PHT, p27, RB und gegen Caspase Aktivatoren, gegen die Matrixmetalloproteinase-2- (MMP-2) und/oder gegen den Aktivator der Matrixmetaloproteinase-1 (MT1-MMP) wirken, oder die weiteren Antikörper monoklonale und/oder polyklonale Antikörper gegen die genannten Antigene sind.

147. Trägermaterial nach einem oder mehreren der Ansprüche 1 bis 146, dadurch gekennzeichnet, daß die weiteren Antikörper gegen HLA-DR 1,2,4, gegen Oligodendrozyten Glykoprotein (MOG), gegen den Inhibitor des Nervenwachstums vom Typ NI 35/250 sowie gegen neurotrophische Stimulatoren und gegen Semaphorin wirken, oder die weiteren Antikörper monoklonale und/oder polyklonale Antikörper gegen die genannten Antigene sind.

148. Trägermaterial nach einem oder mehreren der Ansprüche 1 bis 147, dadurch gekennzeichnet, daß die weiteren Antikörper gegen Wachstumsfaktoren wie Angiogenin, Chemokines, Colony Stimulating Factors (CSF), Epidermal Growth Factors (EGF), Fibroblast Growth Factor (FGF), Hepatocyte Growth Factor (HFG), Insulin.like Growth Factor (IGF), Interferone (INF), Interleukine (IL), Keratinocyte Growth Factor (KGF), Leptin ( OB), Leukemia Inhibitory Factor (LIF) Nerve Growth Factor (NGF), Oncostatin M (OSM), Platelet -Derived Cell Growth Factor (PD-ECGF), Pleiotrophin Stem Cell Factor (SCF), Transforming Growth Factor - a und b (TFG-a, TGF-b) wirken , oder die weiteren Antikörper monoklonale und/oder polyklonale Antikörper gegen die genannten Antigene sind.

149. Trägermaterial nach einem oder mehreren der Ansprüche 1 bis 148, dadurch gekennzeichnet, daß die weiteren Antikörper gegen Reagentien aus der Zellbiologie wie Actin,Cytokeratin, Intermediate Filamente, Mikrotubuline, Zell Adhäsionsproteine, Zellorganellen, Extrazelluläre Matrix, Zellzyklus beeinflussende Proteine, Zellkernstoffwechsel beeinflussende Proteine, humane zelluläre Antigene von Rezeptoren, Hormonen, Signaltransducertypen , Proliferationsproteinen, Superantigenen und Onkogenen sowie deren Vorstufen und Metabolite, gegen Prionen, Viroiden, Viren, Pro- und Eukaryonten, Helminthen, Bakterien Protozoen oder deren Bestandteile wirken , oder die weiteren Antikörper monoklonale und/oder polyklonale Antikörper gegen die genannten Antigene sind.

150. Trägermaterial nach einem oder mehreren der Ansprüche 1 bis 149, dadurch gekennzeichnet, daß der Träger aus Kunststoff, kunststoffbeschichteten Metallen, Glas, Cellulose-verbindungen, Stärke- und Stärkeaggregaten, Sepharose oder Sepharose-Derivaten besteht.

151. Trägermaterial nach Anspruch 150, dadurch gekennzeichnet, daß der Kunststoff Polyolefin, Polyester, Polyether, Polyamid, Polyimid, Polyurethan, Polyvinylchlorid, Polystyrol, Polysulfon, Polyacrylat, Polymethacrylat, Polypropylen, Polyvinylpyrrolidon, ein Fluorpolymeres, ein Derivat dieser Verbindungen oder eine Mischung oder ein Copolymeres davon oder ein Silicon-Kautschuk umfaßt.

152. Trägermaterial nach Anspruch 151, dadurch gekennzeichnet, daß die Kunststoffe polare Eigenschaften besitzen.

153. Trägermaterial nach einen der Ansprüche 151 oder 152, dadurch gekennzeichnet, daß die Kunststoffoberfläche einen Haftvermittler, insbesondere Bromcyan, Thiophosphin und Thionylchlorid aufweist.

154. Trägermaterial nach Anspruch 150 bis 152, dadurch gekennzeichnet, daß die Antikörper mit der Trägeroberfläche kovalent oder adsorptiv daran gebunden sind.

155. Trägermaterial nach einem der Ansprüche 150 bis 154, dadurch gekennzeichnet, daß die Beschichtung die jeweiligen Antikörper gegen TNF und/oder gegen Anti-TNF und/oder gegen Fragmente von TNF und/oder gegen Fragmente von Anti-TNF in einer Menge von 1 bis 95 Gew.-% enthält, und die Restmenge auf 100 Gew.-% aus den weiteren Antikörpern, die gegen spezifische Krankheitserreger wirksam sind, gebildet wird.

156. Trägermaterial nach Anspruch 150 bsi 154, dadurch gekennzeichnet, daß die Beschichtung des Trägers die Antikörper gegen TNF in einer Menge von 1 bis 95 Gew.-% enthält, und die Restmenge auf 100 Gew.-% durch die weiteren Antikörper, die gegen spezifische Krankheitserreger wirksam sind, gebildet wird.

157. Säule zur Blut- bzw. Plasmawäsche, dadurch gekennzeichnet, daß die Säule zur Entfernung krankmachender Antigene mit einem Trägermaterial nach einem oder mehreren der Ansprüche 1 bis 156 ausgerüstet ist.

158. Säule nach Anspruch 157, dadurch gekennzeichnet, daß die Innenwand der Säule mit dem Trägermaterial beschichtet ist.

159. Säule nach Anspruch 157, dadurch gekennzeichnet, daß die Säule mit Trägermaterialkügelchen gefüllt ist.

160. Verwendung der Säule nach einem der Ansprüche 157 bis 159 für den Einsatz bei allen Erkrankungen, bei denen spezifische Erreger, spezifische Wachstumsfaktoren, spezifische Proteine oder Peptide, spezifische Toxine, spezifische Enzyme, spezifische Hormone, spezifische Rezeptoren, spezifische Zell- oder Gewebemarker, spezifische Zytokinine in biologischen Flüssigkeiten, in Geweben und in Zellkulturen von Geweben nachzuweisen sind und zu denen monoklonale Antikörper in definierter Weise herstellbar sind, und deren Antigene oder Antikörper sich zur Beschichtung von extrakorporalen Säulensystemen eignen.

161. Verwendung der Säule nach einem der Ansprüche 157 bis 159 für den Einsatz bei allen Erkrankungen, bei denen spezifische Erreger, spezifische Wachstumsfaktoren, spezifische Proteine und Peptide, spezifische Toxine, spezifische Enzyme, spezifische Hormone, spezifische Rezeptoren, spezifische Zell- oder Gewebemarker, spezifische Zytokinine in biologischen Flüssigkeiten, in Geweben und in Zellkulturen von Geweben nicht nachzuweisen sind, aber im Blut und/oder im Gewebe oder in Zellkulturen von Geweben definierte Antigene und/oder Antikörper, die im direkten oder indirektem Zusammenhang mit den Erkrankungen stehen, meßbar sind, und deren Antigene oder monoklonale Antikörper sich zur Beschichtung von extrakorporalen Säulensystemen eignen.

162. Verwendung der Säule nach einem der Ansprüche 157 bis 159 für den Einsatz bei allen Erkrankungen, bei denen direkt oder indirekt ein Hinweis auf immunogene Prozesse vorliegt und Antigene und/oder Antikörper nachweisbar sind, und deren Antigene oder monoklonale Antikörper sich zur Beschichtung von extrakorporalen Säulensystemen eignen.

163. Verwendung der Säule nach einem der Anspruche 157 bis 159 bei allen Erkrankungen,bei denen andere ursächliche Gründe als die unter Anspruch(bez.Unteranspruch) 1 bis 162 genannten vorliegen,und Isolate,Zellzyklus/Kernstoffwechselproteine aus biologischen Flüssigkeiten von erkrankten Organen sich als Antigene oder monoklonale und/oder polyklonale Antikörper zur Beschichtung oder Füllung von Säulen eignen.
